(19) Europäisches Patentamt / European Patent Office / Office européen des brevets

(11) EP 1 761 234 B1

(12) **EUROPÄISCHE PATENTSCHRIFT**

(45) Veröffentlichungstag und Bekanntmachung des Hinweises auf die Patenterteilung:
**14.08.2013 Patentblatt 2013/33**

(21) Anmeldenummer: **05761658.3**

(22) Anmeldetag: **15.06.2005**

(51) Int Cl.:
***A61K 8/81*** *(2006.01)* ***A61Q 5/02*** *(2006.01)*
***A61Q 5/06*** *(2006.01)* ***A61Q 5/12*** *(2006.01)*
***A61Q 19/00*** *(2006.01)* ***C08F 226/10*** *(2006.01)*

(86) Internationale Anmeldenummer:
**PCT/EP2005/006401**

(87) Internationale Veröffentlichungsnummer:
**WO 2005/123014 (29.12.2005 Gazette 2005/52)**

(54) **WÄSSRIGE ZUBEREITUNGEN ENTHALTEND EIN WASSERLÖSLICHES ODER WASSERDISPERGIERBARES COPOLYMER, DAS WENIGSTENS EIN MONOMER MIT EINEM PROTONIERBAREN STICKSTOFFATOM ENTHÄLT**

AQUEOUS PREPARATIONS COMPRISING A WATER-SOLUABLE OR WATER-DISPERSIBLE COPOLYMER WHICH CONTAINS AT LEAST ONE MONOMER HAVING A NITROGEN ATOM

PREPARATIONS AQUEUSES COMPRENANT UN COPOLYMERE SOLUBLE ET DISPERSIBLE DANS L'EAU QUI CONTIENT AU MOINS UN MONOMERE PRESENTANT UN ATOME D'HYDROGENE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **21.06.2004 DE 102004029773**
**28.02.2005 DE 102005009668**
**02.03.2005 DE 102005010108**

(43) Veröffentlichungstag der Anmeldung:
**14.03.2007 Patentblatt 2007/11**

(73) Patentinhaber: **BASF SE**
**67056 Ludwigshafen (DE)**

(72) Erfinder:
- **PIEROBON, Marianna**
  **67063 Ludwigshafen (DE)**
- **NGUYEN KIM, Son**
  **69502 Hemsbach (DE)**
- **HÖSSEL, Peter**
  **67105 Schifferstadt (DE)**

(74) Vertreter: **Reinhardt, Jürgen et al**
**BASF Personal Care and Nutrition GmbH**
**Postfach 13 01 64**
**40551 Düsseldorf (DE)**

(56) Entgegenhaltungen:
**EP-A- 0 437 114**
**EP-A- 1 302 191**
**WO-A-94/06409**
**DE-A1- 10 151 592**
**DE-A1- 19 738 303**
**US-B1- 6 403 542**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 761 234 B1

## Beschreibung

Die vorliegende Erfindung betrifft wässrige Zubereitungen enthaltend wenigstens ein wasserlösliches oder wasserdispergierbares Copolymer mit kationogenen Gruppen, das wenigstens ein Monomer mit wenigstens einem protonierbaren Stickstoffatom und wenigstens ein weiteres damit copolymerisierbares Monomer einpolymerisiert enthält, und wenigstens einen kosmetisch akzeptablen Träger, wobei der pH-Wert der wässrigen Zubereitung einen Wert im Bereich von pH 4 bis pH 6 aufweist.

Die Erfindung betrifft weiterhin die Verwendung dieser wässrigen Zubereitungen und Verfahren zu ihrer Herstellung.

Kosmetisch und pharmazeutisch akzeptable wasserlösliche Polymere finden in Kosmetik und Medizin vielfache Anwendung. In Seifen, Cremes und Lotionen beispielsweise dienen sie in der Regel als Formulierungsmittel, z.B. als Verdicker, Schaumstabilisator oder Wasserabsorbens oder auch dazu, die reizende Wirkung anderer Inhaltsstoffe abzumildern oder die dermale Applikation von Wirkstoffen zu verbessern.

Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere beispielsweise als Conditioner dazu eingesetzt, um die Trocken- und Nasskämmbarkeit, Anfassgefühl, Glanz und Erscheinungsform zu verbessern sowie dem Haar antistatische Eigenschaften zu verleihen. Es ist bekannt, wasserlösliche Polymere mit kationischen Funktionalitäten in Haarkonditioniermitteln einzusetzen, die eine größere Affinität zur strukturell bedingt negativ geladenen Oberfläche des Haares aufweisen und eine elektrostatische Aufladung des Haares verhindern. Struktur und Wirkungsweise verschiedener Haarbehandlungspolymere sind in Cosmetic & Toiletries 103 (1988) 23 beschrieben. Handelsübliche kationische Conditionerpolymere sind z.B. kationische Hydroxyethylcellulose, kationische Polymere auf der Basis von N-Vinylpyrrolidon, z.B. Copolymere aus N-Vinylpyrrolidon und quarterniertem N-Vinylimidazol oder Copolymere aus Acrylamid und Diallyldimethylammoniumchlorid.

Zur Festigung von Haarfrisuren werden beispielsweise Vinyllactam-Homo- und Copolymere und Carboxylatgruppen-haltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung, möglichst geringe Klebrigkeit des gebildeten Films und ein angenehmer Griff des damit behandelten Haares.

Schwierigkeiten bereitet oft die Bereitstellung von Produkten mit einem komplexen Eigenschaftsprofil. So besteht ein Bedarf an kosmetischen Zubereitungen, die zur Bildung im Wesentlichen glatter, klebfreier Filme befähigt sind, die dem Haar und der Haut gute sensorisch erfassbare Eigenschaften, wie einen angenehmen Griff, verleihen und gleichzeitig eine gute Konditionierwirkung bzw. Festigungswirkung aufweisen.

Die EP-A-670 333 beschreibt vernetzte wasserlösliche Polymerdispersionen, die durch Polymerisation eines Monomergemischs, enthaltend wenigstens ein wasserlösliches Monomer, wenigstens einen Vernetzer sowie gegebenenfalls hydrophobe und/oder amphiphile Monomere in Gegenwart eines polymeren Dispergiermittels erhältlich sind. Als wasserlösliche Monomere können neben einer Vielzahl weiterer auch N-Vinylpyrrolidon sowie Monomere mit kationischen/kationisierbaren Gruppen, wie N-Vinylimidazol, eingesetzt werden.

Die EP-A-929 285 lehrt die Verwendung von wasserlöslichen Copolymeren, die Vinylcarbonsäureamid-Einheiten und Vinylimidazol-Einheiten einpolymerisiert enthalten als Bestandteil kosmetischer Mittel.

Die WO 00/27893 beschreibt wässrige Polymerdispersionen auf der Basis von N-Vinylcarbonsäureamiden und gegebenenfalls Comonomeren, wobei neben einer grossen Anzahl weiterer auch N-Vinylpyrrolidon, N-Vinylimidazol und N-Vinylimidazolderivate genannt werden. Die Polymerisation erfolgt in Gegenwart wenigstens eines polymeren Dispergiermittels.

Die WO 03/92640 betrifft kosmetische Mittel, welche wenigstens ein wasserlösliches Copolymer enthalten, das durch radikalische Copolymerisation von Acrylsäureamid und/oder Methacrylsäureamid und weiteren damit copolymerisierbaren wasserlöslichen a,ß-ethylenisch ungesättigten Verbindungen, gegebenenfalls in Gegenwart einer wasserlöslichen polymeren Pfropfgrundlage, erhältlich sind.

Es besteht nach wie vor Verbesserungsbedarf bei den aus dem Stand der Technik bekannten Zubereitungen für kosmetische und pharmazeutische Anwendungen. Dies gilt insbesondere für Zubereitungen enthaltend Polymere, die neben guten filmbildenden Eigenschaften auch eine Einstellung der rheologischen Eigenschaften der Produkte zulassen, so dass die z.B. in Form von Mousses, Schäumen oder Gelen formuliert werden können.

Aufgabe der vorliegenden Erfindung war es, eine insbesondere für Mousse- und Schaumanwendungen geeignete kosmetische Zubereitung mit verbesserten Festigungseigenschaften zu finden, die sich zudem zur Erzeugung elastischer Frisuren bei gleichzeitig starker Festigung auch bei hoher Luftfeuchtigkeit eignet und sich weiterhin durch gute Auswaschbarkeit, möglichst geringe Klebrigkeit und guten Griff des damit behandelten Haares auszeichnet.

Diese Aufgabe wird gelöst durch wässrige Zubereitungen gemäß Anspruch 1.

Im Rahmen dieser Erfindung sind protonierbare Stickstoffatome solche Stickstoffatome, die durch Protonierung, bevorzugt mit Hilfe von Säuren, in den kationischen Ladungszustand überführt werden können.

Einstellung des pH Wertes

Der pH-Wert Ist ein von Sørensen eingeführter Begriff für den negativen dekadischen Logarithmus der Konzentration der Wasserstoff-Ionen c($H^+$) in mol/L in wässriger Lösung.

Der pH-Wert von Wasser (neutraler pH-Bereich) beträgt 7,0 bei 22°C. Der pH-Wert Ist temperaturabhängig und nimmt zu höheren Temperaturen hin ab. Die Bestimmung des pH-Werts erfolgt Ober dem Fachmann bekannte potentiometrische Messungen mit pH-Elektroden (Glaselektroden) oder kolorimetrisch mit Indikatorfarbstoffen (pH-Papier, Lackmus-Papier, pH-Stäbchen). Beispiele für Indikatoren sind in Ullmann's Encyclopedia of Industrial Chemistry, 6. Auflage, Vol. 17, Seiten 645 bis 655 genannt. Zur Bestimmung des pH-Wertes mit Elektroden können alle kommerziell erhältlichen pH-Messgeräte eingesetzt werden.

Die Bestimmung des pH-Wertes der erfindungsgemäßen Zubereitungen erfolgt nach einer dem Fachmann vorgenannten bekannten Methode bei Temperaturen von 20 bis 25°C.

Erfindungsgemäß weist der pH-Wert der wässrigen Zubereitung einen Wert im Bereich von pH 4 bis pH 6 auf. Bevorzugt wird dieser pH-Wert durch Zugabe einer Brönsted-Säure eingestellt. Bevorzugte Brönsted-Säuren sind wasserlösliche organische und anorganische Säuren.

Als mögliche organische Säuren seien ein- und mehrwertige, gegebenenfalls substituierte aliphatische und aromatische Carbonsäuren, ein- und mehrwertige, gegebenenfalls substituierte aliphatische und aromatische Sulfonsäuren oder ein- und mehrwertige, gegebenenfalls substituierte aliphatische und aromatische Phosphonsäuren.

Bevorzugte organische Säuren sind Hydroxycarbonsäuren, d.h. Derivate der Carbonsäuren, bei denen ein oder mehrere H-Atome durch Hyroxylgruppen ersetzt sind.

Als Beispiele für Hydroxycarbonsäuren seien Glykolsäure, Milchsäure, Weinsäure und Citronensäure genannt.

Bevorzugt wird demnach der pH Wert der Zubereitungen durch Zugabe einer Hydroxysäure eingestellt, wobei Milchsäure besonders bevorzugt ist.

Als bevorzugte anorganische Säuren seien Phosphorsäure, phosphorige Säure, Schwefelsäure, schweflige Säure und Salzsäure genannt.

In einer bevorzugten Ausführungsform weist der pH Wert der wässrigen Zubereitungen einen Wert von mindestens 4.5, bevorzugt 5, besonders bevorzugt 5.2, insbesondere 5.4 und einen Wert von höchstens 6, bevorzugt 5.8, insbesondere 5.6 auf. Erfindungsgemäß kann der pH-Wert bevorzugt aber auch Werte von 5.1, 5.3, 5.5, 5.7 oder 5.9 aufweisen.

Die Einstellung des pH-Wertes der Zubereitungen erfolgt gemäß der Erfindung bevorzugt zu einem Zeitpunkt, zu dem die Herstellung der Komponente A, also die Polymerisation, abgeschlossen ist.

Die Herstellung der Komponente A gilt als abgeschlossen, wenn der Gehalt der Zubereitung an nicht umgesetzten Monomeren weniger als 5, bevorzugt weniger als 2, besonders bevorzugt weniger als 0.1, ganz besonders bevorzugt weniger als 0.05, bezogen auf die Gesamtmasse von Komponente A, beträgt.

Erfindungsgemäß kann die Einstellung des pH Wertes der Zubereitungen zu jedem Zeitpunkt nach abgeschlossener Herstellung der Komponente A erfolgen.

Zusätzlich zur erfindungsgemäßen Einstellung des pH-Wertes der wässrigen Zubereitung nach abgeschlossener Herstellung der Komponente A auch die pH-Werte der Monomerlösungen vor oder während der Herstellung der Komponente A auf Werte im Bereich von pH 6 bis pH 7 eingestellt werden.

Es kann demnach auch von Vorteil sein, die pH-Werte der Monomer-Zuläufe vor oder während der Polymerisation auf einen Wert im Bereich von pH 6 bis pH 7 einzustellen und den pH-Wert der wässrigen Zubereitung nach abgeschlossener Polymerisation erfindungsgemäß auf einen Wert von pH 4 bis pH 6 einzustellen.

Ein weiterer Gegenstand der Erfindung ist demnach ein Verfahren zur Herstellung der erfindungsgemäßen Zubereitung, dadurch gekennzeichnet, dass die Einstellung des pH Wertes auf einen Wert im Bereich von 4 bis 6 nach abgeschlossener Herstellung von Komponente A) vorgenommen wird.

In einer besonders bevorzugten Ausführungsform wird der pH-Wert der Zubereitung dann eingestellt, wenn die Zubereitung bereits in ihrer Anwendungsform, beispielsweise als Gel-, Schaum-, Spray-, Salben-, Creme-, Emulsions-, Suspensions-, Lotions-, Milch- oder Pastenzubereitungen vorliegt.

Erfindungsgemäß erfolgt also die Einstellung des pH Wertes bevorzugt an kosmetisch akzeptablen Gel-, Schaum-, Spray-, Salben-, Creme-, Emulsions-, Suspensions-, Lotions-, Milch- oder Pastenzubereitungen.

Im Rahmen der vorliegenden Erfindung umfasst der Ausdruck Alkyl geradkettige und verzweigte Alkylgruppen. Erfindungsgemäß geeignete kurzkettige Alkylgruppen sind z.B. geradkettige oder verzweigte $C_1$-$C_7$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc..

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen zB. n-Hexyl(en), n-Heptyl(en), n-Octyl(en), n-Nonyl (en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Cycloalkyl steht vorzugsweise für $C_5$-$C_8$-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure, und Methacrylsäure ableiten können teilweise verkürzt durch Einfügen der Silbe "(meth)" in die von der Acrylsäure abgeleitete Verbindung bezeichnet

Die erfindungsgemäßen Zubereitungen lassen sich unter Normalbedingungen vorteilhaft als Schaum formulieren.

Unter wasserlöslichen Monomeren und Polymeren werden im Rahmen der vorliegenden Erfindung Monomere und Polymere verstanden, die sich zu mindestens 1 g/l bei 20°C in Wasser lösen.

Unter wasserdispergierbaren Monomeren und Polymeren werden Monomere und Polymere verstanden, die unter Anwendung von Scherkräften, beispielsweise durch Rühren, in dispergierbare Partikel zerfallen.

Hydrophile Monomere sind vorzugsweise wasserlöslich oder zumindest wasserdispergierbar.

Zur Herstellung der Copolymere A) werden Monomere mit wenigstens einem protonierbaren Stickstoffatom, insbesondere N-Vinylimidazol und/oder dessen Derivate eingesetzt. Das Copolymer A) enthält demnach wenigstens ein Monomer mit wenigstens einem protonierbaren Stickstoffatom ausgewählt aus N-Vinylimidazol und/oder eines Derivates davon

in nicht quaternisierter Form.

Unter "quaternisierter Form" wird in diesem Zusammenhang der kationische Ladungszustand des Stickstoffatoms verstanden, der beispielsweise durch Alkylierung aber nicht durch Protonierung erzeugt wird.

Es ist auch möglich, zur Herstellung der Copolymere A) weitere, von ungeladenen N-Vinylimidazol(derivaten) verschiedene geladene N-Vinylimidazol(derivate) einzusetzen. Desweiteren ist es möglich, von N-Vinylimidazol(derivaten) verschiedene, weitere kationogene und/oder kationische Monomere (d.h. weitere Monomere in nicht, teilweise oder vollständig protonierter und/oder quaternisierter Form) einzusetzen.

Bevorzugt enthalten die Copolymere A) keine Monomeren mit anionogenen und/oder anionischen Gruppen einpolymerisiert.

Monomer a)

Das in den erfindungsgemäßen Zubereitungen eingesetzte Copolymer A) enthält vorzugsweise 0.5 bis 40 Gew.-%, besonders bevorzugt 1 bis 30 Gew.-%, ganz besonders bevorzugt 3 bis 20 Gew.-%, und Insbesondere 3 bis 15 Gew.-%, bezogen auf das Gesamtgewicht der zur Polymerisation eingesetzten Monomere, wenigstens eines Monomers a) einpolymerisiert. In einer speziellen Ausführungsform beträgt der Anteil an Monomeren a) höchstens 25 Gew.-%.

Das in den erfindungsgemäßen Zubereitungen eingesetzte Copolymer A) enthält wenigstens ein Monomer mit wenigstens einem protonierbaren Stickstoffatom einpolymerisiert.

Das Copolymer A) enthält als Monomer a) wenigstens eine N-Vinylimidazol-Verbindung der allgemeinen Formel (1)

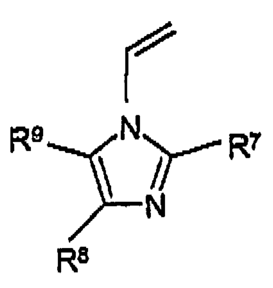

(I)

einpolymerisiert, worin $R^7$ bis $R^9$ unabhängig voneinander für Wasserstoff, $C_1$-$C_4$-Alkyl oder Phenyl stehen.

Beispiele für Verbindungen der allgemeinen Formel (I) sind folgender Tabelle 1 zu entnehmen:

Tabelle 1

| $R^7$ | $R^8$ | $R^9$ |
|---|---|---|
| | | |
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |
| Me = Methyl<br>Ph = Phenyl | | |

Ganz besonders bevorzugtes Monomer a) ist 1-Vinylimidazol (N-Vinylimidazol).

Monomer b)

Das in den erfindungsgemäßen Zubereitungen eingesetzte Copolymer A) enthält vorzugsweise 20 bis 99.5 Gew.-%, besonders bevorzugt 20 bis 70 Gew.-%, insbesondere 30 bis 70 Gew.-%, bezogen auf das Gesamtgewicht der zur Polymerisation eingesetzten Monomere, wenigstens eines weiteren, damit copolymerisierbaren Monomers b) einpolymerisiert. In einer speziellen Ausführungsform beträgt der Anteil an Monomeren b) mindestens 50 Gew.-%.

Monomer b1)

Das Copolymer A) enthält zusätzlich wenigstens ein N-Vinyllactam b1) einpolymerisiert. Als Monomere b1) eignen sich unsubstituierte N-Vinyllactame und N-Vinyllactamderivate, die z.B. einen oder mehrere $C_1$-$C_6$-Alkylsubstituenten, wie Methyl, Ethyl, n-Propyl, Isopropyl, n-Butyl, sec.-Butyl, tert.-Butyl etc., aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. sowie Mischungen daraus.

Bevorzugt werden N-Vinylpyrrolidon, N-Vinylcaprolactam oder Mischungen daraus eingesetzt.

In einer speziellen Ausführungsform enthalten die erfindungsgemäßen Zubereitungen als Komponente A) ein Copolymer, das nur aus Monomereinheiten der zuvorgenannten Monomere a) und b1) besteht

Bevorzugt enthalten diese Copolymere A) dann 0.5 bis 40 Gew.-%, besonders bevorzugt 1 bis 30 Gew.-%, insbesondere 3 bis 20 Gew.-% wenigstens eines Monomers a) einpolymerisiert. Entsprechend enthalten diese Copolymere A) bevorzugt 60 bis 99.5 Gew.-%, besonders bevorzugt 70 bis 99 Gew.-%, insbesondere 80 bis 97 Gew.-% wenigstens eines Monomers b) einpolymerisiert.

In einer bevorzugten Ausführungsform enthalten die erfindungsgemäßen Zubereitungen als Komponente A)

ein Copolymer, das zusätzlich zu den zuvorgenannten Monomeren a) und b1) wenigstens ein weiteres, davon verschiedenes Monomer b2) einpolymerisiert enthält.

Monomer b2)

Die Copolymere A) können zusätzlich wenigstens ein von den Komponenten a) und b1) verschiedenes, damit copolymerisierbares nichtionisches wasserlösliches Monomer b2) einpolymerisiert enthalten.
Vorzugsweise beträgt der Anteil an Monomeren b2) 0 bis 50 Gew.-%, besonders bevorzugt 5 bis 50 Gew.-%, insbesondere 10 bis 40 Gew.-%, bezögen auf das Gesamtgewicht der zur Polymerisation eingesetzten Monomere.
Vorzugsweise ist die Komponente b2) ausgewählt unter

b2.1) N-Vinylamiden gesättigter $C_1$-$C_8$-Monocarbonsäuren,

b2.2) primären Amiden $\alpha,\beta$-ethylenisch ungesättigter Monocarbonsäuren und deren N-Alkyl- und N,N-Dialkylderivaten, die zusätzlich zu dem Carbonyl-Kohlenstoffatom der Amidgruppe höchstens 8 weitere Kohlenstoffatome aufweisen,

b2.3) Estern $\alpha,\beta$-ethylenisch ungesättigter Mono- und Dicarbonsäuren mit Diolen,

b2.4) Amiden $\alpha,\beta$-ethylenisch ungesättigter Mono- und Dicarbonsäuren mit Aminoalkoholen, die eine primäre oder sekundäre Aminogruppe aufweisen,

b2.5) Polyetheracrylaten

und Mischungen davon.
Als Monomere b2.1) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.
Geeignete Monomere b2.2) sind beispielsweise Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N, N-Dimethyl(meth)acrylamid, N,N-Diethyl(meth)acrylamid Piperidinyl(meth)acrylamid und Morpholinyl(meth)acrylamid, wobei (Meth)acrylsäureamid bevorzugt und Methacrylsäureamid besonders bevorzugt ist.
Geeignete Monomere b2.3) sind beispielsweise 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.
Geeignete Monomere b2.4) sind beispielsweise 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.
Geeignete Monomere b2.5) sind Polyetheracrylate, worunter im Rahmen dieser Erfindung allgemein Ester $\alpha$, $\beta$-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20 000 auf. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z.B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.
Bevorzugt als Komponente b2.5) sind Polyetheracrylate der allgemeinen Formel V

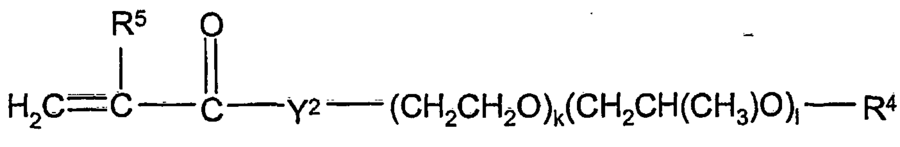

(V)

worin
die Reihenfolge der Alkylenoxideinheiten beliebig ist,
k und l unabhängig voneinander für eine ganze Zahl von 0 bis 1000 stehen, wobei die Summe aus k und I mindestens 5 beträgt,

$R^4$ für Wasserstoff, $C_1$-$C_{30}$-Alkyl oder $C_5$-$C_8$-Cycloalkyl steht,

$R^5$ für Wasserstoff oder $C_1$-$C_8$-Alkyl steht,

$Y^2$ für O oder $NR^6$ steht, wobei $R^6$ für Wasserstoff, $C_1$-$C_{30}$-Alkyl oder $C_5$-$C_8$-Cycloalkyl steht,

Bevorzugt steht k für eine ganze Zahl von 1 bis 500, insbesondere 3 bis 250. Bevorzugt steht I für eine ganze Zahl von 0 bis 100.

Bevorzugt steht $R^5$ für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht $R^4$ in der Formel IV für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl.

Vorzugsweise steht $Y^2$ in der Formel IV für O oder NH.

Geeignete Polyetheracrylate b2.5) sind z.B. die Polykondensationsprodukte der zuvor genannten $\alpha,\beta$-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol $R^4$-OH hergestellt werden. Die Alkylenoxide können einzeln, alternierend nacheinander oder als Mischung eingesetzt werden. Die Polyetheracrylate c) können allein oder in Mischungen zur Herstellung der erfindungsgemäß eingesetzten Polymere verwendet werden.

Geeignete Polyetheracrylate b2.5) sind auch Urethan(meth)acrylate mit Alkylenoxidgruppen. Derartige Verbindungen sind in der DE 198 38 851 (Komponente e2)) beschrieben, worauf hier in vollem Umfang Bezug genommen wird.

Die zuvor genannten Monomere b2) können jeweils einzeln oder in Form von beliebigen Mischungen eingesetzt werden.

In einer bevorzugten Ausführungsform der Erfindung enthalten die Zubereitungen als Komponente A ein Terpolymer, das 5 bis 15 Gew.-% Monomer a), 30 bis 70 Gew.-% Monomer b1) und 20 bis 35 Monomer b2) einpolymerisiert enthält, mit der Maßgabe, dass die Summe der Mengen der Monomere a) bis b2) 100 Gew.-% ergibt.

Monomer b3)

Die Copolymere A) können zusätzlich wenigstens ein von a), b1) und b2) verschiedenes wasserlösliches Monomer b3) einpolymerisiert enthalten, das ausgewählt ist unter $\alpha,\beta$-ethylenisch ungesättigten wasserlöslichen Verbindungen mit kationischen hydrophilen Gruppen.

Vorzugsweise beträgt der Anteil an Monomer b3) 0 bis 30 Gew.-%, besonders bevorzugt 0 bis 20 Gew.-%, insbesondere 0 bis 10 Gew.-%, bezogen auf das Gesamtgewicht der zur Polymerisation eingesetzten Monomere.

Bevorzugt handelt es sich bei den kationischen Gruppen der Komponente b3) um stickstoffhaltige Gruppen wie quaternäre Ammoniumgruppen.

Diese geladenen kationischen Gruppen lassen sich aus den Aminstickstoffen durch Quaternisierung, z.B. mit den zuvor bei der Komponente a) genannten Alkylierungsmitteln erzeugen. Beispiele für Alkylierungsmittel sind $C_1$-$C_4$-Alkylhalogenide oder -sulfate, wie Ethylchlorid, Ethylbromid, Methylchlorid, Methylbromid, Dimethylsulfat und Diethylsulfat. Eine Quaternisierung kann im Allgemeinen sowohl vor als auch nach der Polymerisation erfolgen.

Geeignete Monomere b3) sind die durch Quaternisierung der Komponente a) erhältlichen Verbindungen. Beispiele für solche geladenen Monomere b3) sind quaternisierte N-Vinylimidazole, insbesondere 3-Methyl-1-vinylimidazoliumchlorid und -methosulfat.

Geeignete Verbindungen b3) sind weiterhin die Quaternisierungsprodukte der Ester von $\alpha,\beta$-ethylenisch ungesättigten Mono- und Dicarbonsäuren mit Aminoalkoholen. Bevorzugte Aminoalkohole sind $C_2$-$C_{12}$-Aminoalkohole, welche am Aminstickstoff $C_1$-$C_8$-dialkyliert sind. Als Säurekomponente dieser Ester eignen sich z.B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere b3) sind die Quaternisierungsprodukte von N,N-Dimethylaminomethyl(meth)acrylat, N, N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete Monomere b3) sind weiterhin die Quaternisierungsprodukte der Amide der zuvor genannten $\alpha,\beta$-

ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Geeignet als Monomere b3) sind z.B. die Quaternisierungsprodukte von N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl] acrylamid, N-[4-(dirnethylamino)cyclohexyl]acrylamid und N-[4-(dimethylamino)cyclohexyl]methacrylamid.

Geeignete Monomere b3) sind weiterhin die Quaternisierungsprodukte der N,N-Diallylamine und N,N-Diallyl-N-alkylamine. Alkyl steht dabei vorzugsweise für $C_1$-$C_{24}$-Alkyl. Bevorzugt sind N,N-Diallyl-N,N-dimethylammonium-Verbindungen, wie z.B. die Chloride und Bromide. Dazu zählt insbesondere N,N-Diallyl-N,N-dimethylammoniumchlorid (DADMAC).

Geeignete Monomere b3) sind weiterhin die Quaternisierungsprodukte verschiedener vinyl- und allylsubstituierte Stickstoffheterocyclen, wie 2- und 4-Vinylpyridin, 2- und 4-Allylpyridin.

Die zuvor genannten Monomere b3) können jeweils einzeln oder in Form von beliebigen Mischungen eingesetzt werden.

In einer bevorzugten Ausführungsform der Erfindung enthalten die Zubereitungen als Komponente A ein Polymer, das 5 bis 15 Gew.-% Monomer a), 30 bis 70 Gew.-% Monomer b1), 20 bis 35 Gew.-% Monomer b2) und 0 bis 10 Gew.-% Monomer b3) einpolymerisiert enthält, mit der Maßgabe, dass die Summe der Mengen der Monomere a) bis b3) 100 Gew.-% ergibt.

Monomer c)

Die Copolymere A) können zusätzlich wenigstens ein von den Monomeren a) bis b3) verschiedenes Monomer c) einpolymerisiert enthalten. Die zusätzlichen Monomere c) sind vorzugsweise ausgewählt unter Estern $\alpha,\beta$-ethylenisch ungesättigter Mono- und Dicarbonsäuren mit $C_1$-$C_{30}$-Alkanolen, N-Alkyl- und N,N-Dialkylamiden $\alpha,\beta$-ethylenisch ungesättigter Monocarbonsäuren, die zusätzlich zu dem Carbonyl-Kohlenstoffatom der Amidgruppe mindestens 9 weitere Kohlenstoffatome aufweisen, Estern von Vinylalkohol und Allylalkohol mit $C_1$-$C_{30}$-Monocarbonsäuren, Vinylethern, Vinylaromaten, Vinylhalogeniden, Vinylidenhalogeniden, $C_1$-$C_8$-Monoolefinen, nicht aromatischen Kohlenwasserstoffen mit mindestens zwei konjugierten Doppelbindungen und Mischungen davon.

Vorzugsweise beträgt der Anteil an Monomeren c) 0 bis 15 Gew.%, besonders bevorzugt 0,1 bis 10 Gew.-%, bezogen auf das Gesamtgewicht der zur Polymerisation eingesetzten Monomere.

Geeignete zusätzliche Monomere c) sind Methyl(meth)acrylat, Methylethacrylat, Ethyl(meth)acrylat, Ethylethacrylat, n-Butyl(meth)acrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth) acrylat, Melissinyl(meth)acrylat, Palmitoleinyl(meth)acrylat, Oleyl(meth)acrylat, Linolyl(meth)acrylat, Linolenyl(meth) acrylat, Stearyl(meth)acrylat, Lauryl(meth)acrylat und Mischungen davon. Bevorzugte Monomere c) sind die Ester $\alpha,\beta$-ethylenisch ungesättigter Mono- und Dicarbonsäuren mit $C_1$-$C_4$-Alkanolen.

Geeignete zusätzliche Monomere c) sind weiterhin N-(n-Octyl)(meth)acrylamid, N-(1,1,3,3-Tetramethylbutyl) (meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl (meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl (meth)acrylamid, N-Lauryl(meth)acrylamid.

Geeignete zusätzliche Monomere c) sind weiterhin Vinylacetat, Vinylpropionat, Vinylbutyrat und Mischungen davon.

Geeignete zusätzliche Monomere c) sind weiterhin Ethylen, Propylen, Isobutylen, Butadien, Styrol, $\alpha$-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere c) können einzeln oder in Form von beliebigen Mischungen eingesetzt werden.

Vernetzer d)

Die Copolymere A) können gewünschtenfalls wenigstens einen Vernetzer, d.h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 3 Gew.-%, besonders bevorzugt 0,1 bis 2 Gew.-

%, bezogen auf das Gesamtgewicht der zur Polymerisation eingesetzten Monomere, verwendet.

Geeignete Vernetzer d) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis (hydroxymethyl)cyclohexan, Hydroxypivalinsäure-neopentylglykolmonoester, 2,2-Bis(4-hydroxyphenyl)-propan, 2,2-Bis [4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer d) sind die Vinylester oder die Ester einwertiger, ungesättigter Alkohole mit ethylenisch ungesättigten $C_3$-$C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer d) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer d) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer d) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer d) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Weitere geeignete Vernetzer d) sind Divinyldioxan, Tetraallylsilan oder Tetravinylsilan.

Selbstverständlich können auch Mischungen der vorgenannten Verbindungen d) eingesetzt werden. Vorzugsweise werden wasserlösliche Vernetzer d) eingesetzt.

Besonders bevorzugt eingesetzte Vernetzer d) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer d) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Bevorzugt sind Zubereitungen, wobei das Copolymer A)

a) N-Vinylimidazol und/oder ein Derivat davon und

b1) wenigstens ein N-Vinyllactam

b2) gegebenfalls wenigstens ein nichtionisches wasserlösliches Monomer ausgewählt aus

b2.1) N-Vinylamiden gesättigter $C_1$-$C_8$-Monocarbonsäuren,

b2.2) primären Amiden α,β-ethylenisch ungesättigter Monocarbonsäuren und deren N-Alkyl- und N,N-Dialkylderivaten, die zusätzlich zu dem Carbonyl-Kohlenstoffatom der Amidgruppe höchstens 8 weitere Kohlenstoffatome aufweisen,

b3) gegebenenfalls wenigstens ein Monomer ausgewählt aus den Quaternisierungsprodukten des N-Vinylimidazols und Dimethylaminopropylmethacrylsäureamids.

einpolymerisiert enthält, wobei der pH-Wert der wässrigen Zubereitung einen Wert im Bereich von pH 4 bis pH 6 aufweist.

Bevorzugt sind weiterhin Zubereitungen, wobei das Copolymer A)

a) 0.5 bis 40 Gew.-% N-Vinylimidazol und/oder eines Derivats davon,

b1) 20 bis 99.5 Gew.-% wenigstens eines N-Vinyllactams,

b2) 0 bis 50 Gew.-% wenigstens eines von den Komponenten a) und b1) verschiedenen, damit copolymerisierbaren nichtionischen wasserlöslichen Monomers, und

b3) 0 bis 30 Gew.-% wenigstens eines Monomers, das ausgewählt unter α,β-ethylenisch ungesättigten wasserlöslichen Verbindungen mit kationischen hydrophilen Gruppen, mit der Maßgabe, dass die Summe der Mengen der Komponenten a) bis b3) 100 Gew.-% ergibt,

einpolymerisiert enthält, wobei der pH-Wert der wässrigen Zubereitung einen Wert im Bereich von pH 4 bis pH 6 aufweist.

Desweiteren bevorzugt sind Zubereitungen, wobei das Copolymer A)

a) 1 bis 30 Gew.-% N-Vinylimidazol und/oder eines Derivats davon,

b1) 20 bis 70 Gew.-% wenigstens eines N-Vinyllactams,

b2) 5 bis 50 Gew.-% wenigstens eines von den Komponenten a) und b1) verschiedenen, damit copolymerisierbaren nichtionischen wasserlöslichen Monomers, und

b3) 0 bis 20 Gew.-% wenigstens eines Monomers, das ausgewählt unter α,β-ethylenisch ungesättigten wasserlöslichen Verbindungen mit kationischen hydrophilen Gruppen, mit der Maßgabe, dass die Summe der Mengen der Komponenten a) bis b3) 100 ergibt,

einpolymerisiert enthält, wobei der pH-Wert der wässrigen Zubereitung einen Wert im Bereich von pH 4 bis pH 6 aufweist.

Desweiteren bevorzugt sind Zubereitungen, wobei das Copolymer A)

a) 3 bis 20 Gew.-% N-Vinylimidazol und/oder eines Derivats davon,

b1) 30 bis 70 Gew.-% wenigstens eines N-Vinyllactams,

b2) 10 bis 40 Gew.-% wenigstens eines von den Komponenten a) und b1) verschiedenen, damit copolymerisierbaren nichtionischen wasserlöslichen Monomers, und

b3) 0 bis 10 Gew.-% wenigstens eines Monomers, das ausgewählt unter α,β-ethylenisch ungesättigten wasserlöslichen Verbindungen mit kationischen hydrophilen Gruppen, mit der Maßgabe, dass die Summe der Mengen der Komponenten a) bis b3) 100 Gew.-% ergibt,

einpolymerisiert enthält, wobei der pH-Wert der wässrigen Zubereitung einen Wert im Bereich von pH 4 bis pH 6 aufweist.

In einer bevorzugten Ausführungsform besteht das Copolymer A) nur aus Wiederholungseinheiten, die sich von den zuvor genannten Monomeren a), b1) und gegebenenfalls b2) und/oder b3) ableiten.

In einer besonders bevorzugten Zubereitung enthält Copolymer A)

a) 3 bis 15 Gew.-% N-Vinylimidazol
b) 30 bis 70 Gew.-% N-Vinylpyrrolidon
c) 20 bis 35 Gew.-% Methacrylsäureamid
d) 0 bis 10 Gew.-% quaterniertes N-Vinylimidazol

mit der Maßgabe, dass die Summe der Mengen der Komponenten a) bis b3) 100 Gew.-% ergibt, einpolymerisiert.

Die Herstellung der Copolymere A) erfolgt nach üblichen, dem Fachmann bekannten Verfahren, z.B. durch Lösungs-, Fällungs-, Suspensions- oder Emulsionspolymerisation. Bevorzugt ist die Herstellung durch Lösungs- oder Fällungspolymerisation.

Bevorzugte Lösemittel zur Lösungspolymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan. Besonders bevorzugt ist die Polymerisation in Wasser oder einem Wasser/Alkohol-Gemisch, beispielsweise in einem Wasser/ Ethanol-Gemisch.

Bei der Verwendung von Wasser als Lösungsmittel-Bestandteil wird bevorzugt entsalztes Wasser eingesetzt.

Die Fällungspolymerisation erfolgt beispielsweise in einem Ester, wie Essigsäureethylester oder Essigsäurebutylester als Lösungsmittel. Die resultierenden Polymerteilchen fallen aus der Reaktionslösung aus und können durch übliche Verfahren, wie Filtration mittels Unterdruck, isoliert werden. Bei der Fällungspolymerisation werden in der Regel Polymere mit höheren Molekulargewichten als bei der Lösungspolymerisation erhalten.

Die Polymerisationstemperaturen liegen vorzugsweise in einem Bereich von etwa 30 bis 120°C, besonders bevorzugt 40 bis 100°C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate A) können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-amidinopropan)hydrochloride (V50 von Wako Pure Chemicals Industries, Ltd.), oder 2,2'-Azobis(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, $H_2O_2$/Cu'.

Zur Einstellung des Molekulargewichts kann die Polymerisation in Gegenwart wenigstens eines Reglers erfolgen. Als Regler können die üblichen, dem Fachmann bekannten Verbindungen, wie z.B. Schwefelverbindungen, z.B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, gegebenenfalls bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Erfindungsgemäß kann die Einstellung des pH-Wertes der wässrigen Zubereitung auf einen Wert im Bereich von pH 4 bis pH 6 vor oder nach des Wasserdampfstrippens oder der Wasserdampfdestillation durchgeführt werden.

Wird bei der Herstellung der Polymere ein organisches Lösungsmittel eingesetzt, so kann dieses durch übliche, dem Fachmann bekannte Verfahren, z.B. durch Destillation bei vermindertem Druck, entfernt werden.

Die Polymerisation erfolgt vorzugsweise bei einem pH-Wert im Bereich von 6 bis 9, besonders bevorzugt von 6 bis 7,5. Die Einstellung des pH-Wertes erfolgt durch Zugabe einer geeigneten Säure oder durch Zugabe einer geeigneten Base.

Die erhaltenen flüssigen Polymerzusammensetzungen können durch verschiedene Trocknungsverfahren, wie z.B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige

Copolymere haben den Vorteil einer besseren Lagerfähigkeit, einer einfacheren Transportmöglichkeit und zeigen in der Regel eine geringere Neigung für Keimbefall.

Ein weiterer Gegenstand der Erfindung ist es, die flüssigen Polymerzusammensetzungen nach abgeschlossener Polymerisation und vor der Trocknung und/oder Überführung in die Pulverform auf einen pH-Wert im Bereich von pH 4 bis pH 6 einzustellen.

Kosmetisch akzeptabler Träger B)

Die erfindungsgemäßen Zubereitungen weisen einen kosmetisch und/oder pharmazeutisch akzeptablen Träger B) auf, der ausgewählt ist unter

i) Wasser,

ii) wassermischbaren organischen Lösungsmitteln, vorzugsweise $C_2$-$C_4$-Alkanolen, insbesondere Ethanol,

iii) Ölen, Fetten, Wachsen,

iv) von iii) verschiedenen Estern von $C_6$-$C_{30}$-Monocarbonsäuren mit ein-, zwei- oder dreiwertigen Alkoholen,

v) gesättigten acyclischen und cyclischen Kohlenwasserstoffen,

vi) Fettsäuren,

vii) Fettalkoholen,

viii) Treibgasen,

und Mischungen davon.

Die erfindungsgemäßen Zubereitungen weisen z.B. eine Öl- bzw. Fettkomponente B) auf, die ausgewählt ist unter: Kohlenwasserstoffen geringer Polarität wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z.B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie $C_1$-$C_{10}$-Salicylaten, z.B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle B) sind z.B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z.B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen Zubereitungen kann es sich um hautkosmetische, haarkosmetische, dermatologische, hygienische oder pharmazeutische Zubereitungen handeln. Aufgrund ihrer filmbildenden Eigenschaften eignen sich die zuvor beschriebenen Zubereitungen insbesondere für Haar- und Hautkosmetika.

Vorzugsweise liegen die erfindungsgemäßen Zubereitungen in Form eines Gels, Schaums, Sprays, einer Mousse, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Zubereitungen können zusätzlich kosmetisch und/oder dermatologisch aktive Wirkstoffe sowie Hilfsstoffe enthalten.

Vorzugsweise enthalten die erfindungsgemäßen kosmetischen Zubereitungen wenigstens ein wie vorstehend definiertes Copolymer A), wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z.B. Carboxymethylcellulose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

Bevorzugt enthalten die erfindungsgemäßen Zubereitungen zusätzlich wenigstens ein nichtionisches Verdikkungsmittel.

Geeignete kosmetisch und/oder dermatologisch aktive Wirkstoffe sind z.B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z.B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z.B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z.B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind z.B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z.B. 2-Ethyl-1,3-hexandiol, N,N-Diethyl-m-toluamid etc. Geeignete hyperemisierend wirkende Stoffe, welche die Durchblutung der Haut anregen, sind z.B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z.B. Salicylsäure, Kalziumthioglykolat, Thioglykolsäure und ihre Salze, Schwefel, etc. Geeignete Antischuppen-Wirkstoffe sind z.B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z.B. Allantoin, Bisabolol, Dragosantol, Kamillenextrakt, Panthenol, etc.

Die erfindungsgemäßen kosmetischen Zubereitungen können als kosmetischen und/oder pharmazeutischen Wirkstoff (wie auch gegebenenfalls als Hilfsstoff) wenigstens ein weiteres kosmetisch oder pharmazeutisch akzeptables Polymer enthalten. Dazu zählen ganz allgemein kationische, amphotere und neutrale Polymere.

Die erfindungsgemäßen wässrigen Zubereitungen können weiterhin von Copolymer A) unterschiedliche wasserlösliche Polymere enthalten.

Geeignete Polymere sind z.B. kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care, Luviquat® UltraCare), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z.B. Guarpolymere, wie die Jaguar®-Marken der Fa. Rhodia.

Weitere geeignete Polymere sind auch neutrale Polymere, wie Polyvinylpyrrolidone, Copolymere aus N-Vinylpyrrolidon und Vinylacetat und/oder Vinylpropionat und/oder Stearyl(meth)acrylat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF) oder Kollicoat® IR.

Geeignete Polymere sind auch die in der WO 03/092640 beschriebenen, insbesondere die als Beispiele 1 bis 50 (Tabelle 1, Seite 40 ff.) und Beispiele 51 bis 65 (Tabelle 2, Seite 43) beschriebenen (Meth)Acrylsäureamidcopolymere, auf die an dieser Stelle vollumfänglich Bezug genommen wird.

Geeignete Polymere sind auch nichtionische, wasserlösliche bzw. wasserdispergierbare Polymere oder Oligomere, wie Polyvinylcaprolactam, z.B. Luviskol® Plus (BASF), oder Polyvinylpyrrolidon und deren Copolymere, insbesondere mit Vinylestern, wie Vinylacetat, z.B. Luviskol® VA 37 (BASF); Polyamide, z.B. auf Basis von Itaconsäure und aliphatischen Diaminen, wie sie z.B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z.B. Polyethersiloxane, wie Tegopren® (Fa. Goldschmidt) oder Belsil® (Fa. Wacker).

Außerdem geeignet sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaluronsäure- und RNA-Derivate.

Weitere erfindungsgemäße Zubereitungen enthalten wenigstens ein weiteres wasserlösliches Polymer, insbesondere Chitosane (Poly(D-glucosamine)) verschiedenen Molekulargewichtes und/oder Chitosanderivate.

Anionische Polymere

Weitere für die erfindungsgemäßen Zubereitungen geeignete Polymere sind Carbonsäuregruppen-haltige Copolymere. Dabei handelt es sich um Polyelektrolyte mit einer größeren Anzahl anionisch dissoziierbarer Gruppen in der Hauptkette und/oder einer Seitenkette. Sie sind befähigt, mit den Copolymeren A) Polyelektrolyt-Komplexe (Symplexe) zu bilden.

In einer bevorzugten Ausführungsform weisen die in den erfindungsgemäßen Mitteln eingesetzten Polyelektrolytkomplexe einen Überschuß an anionogenen/anionischen Gruppen auf.

Die Polyelektrolytkomplexe umfassen neben wenigstens einem der zuvor genannten Copolymere A) auch wenigstens ein Säuregruppen-haltiges Polymer.

Die Polyelektrolyt-Komplexe enthalten vorzugsweise Copolymer(e) A) und Säuregruppen-haltige Polymere in einem Gewichtsmengenverhältnis von etwa 50:1 bis 1:20, besonders bevorzugt von 20:1 bis 1:5.

Geeignete Carbonsäuregruppen-haltige Polymere sind z.B. durch radikalische Polymerisation $\alpha,\beta$-ethylenisch ungesättigter Monomere erhältlich. Dabei werden Monomere m1) eingesetzt, die wenigstens eine radikalisch polymerisierbare, $\alpha,\beta$-ethylenisch ungesättigte Doppelbindung und wenigstens eine anionogene und/oder anionische Gruppe pro Molekül enthalten.

Geeignete Carbonsäuregruppenhaltige Polymere sind weiterhin Carbonsäuregruppen-haltige Polyurethane.

Vorzugsweise sind die Monomere ausgewählt unter monoethylenisch ungesättigten Carbonsäuren, Sulfonsäuren, Phosphonsäuren und Mischungen davon.

Zu den Monomeren m1) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren zählen weiterhin die Halbester von monoethylenisch ungesättigten Dicarbonsäuren mit 4 bis 10 vorzugsweise 4 bis 6 C-Atomen, z.B. von Maleinsäure wie Maleinsäuremonomethylester. Zu den Monomeren zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren zählen auch die Salze der zuvor genannten Säuren, insbesondere die Natrium-, Kalium- und Ammoniumsalze sowie die Salze mit den zuvor genannten Aminen. Die Monomere können als solche oder als Mischungen untereinander eingesetzt werden. Die angegebenen Gewichtsanteile beziehen sich sämtlich auf die Säureform.

Vorzugsweise wird das Monomer m1) ausgewählt unter Acrylsäure, Methacrylsäure, Ethacrylsäure, $\alpha$,-Chloracrylsäure, Crotonsäure, Maleinsäure, Maleinsäureanhydrid, Fumarsäure, Itaconsäure, Citraconsäure, Mesaconsäure, Glutacorisäure, Aconitsäure und Mischungen davon, besonders bevorzugt Acrylsäure, Methacrylsäure und Mischungen davon.

Die zuvor genannten Monomere m1) können jeweils einzeln oder in Form von beliebigen Mischungen eingesetzt werden.

Prinzipiell eignen sich als Comonomere zur Herstellung der Carbonsäuregruppen-haltigen Polymere die zuvor als Komponenten des Copolymers A) genannten Verbindungen a) bis d) mit der Maßgabe, dass der Molanteil an anionogenen und anionischen Gruppen, die das Carbonsäuregruppen-haltige Polymer einpolymerisiert enthält, größer ist als der Molanteil an kationogenen und kationischen Gruppen.

In einer bevorzugten Ausführung enthalten die Carbonsäuregruppen-haltigen Polymere wenigstens ein Monomer einpolymerisiert, das ausgewählt ist unter den zuvor genannten Vernetzern d). Auf geeignete und bevorzugte Vernetzer d) wird Bezug genommen.

Des Weiteren bevorzugt enthalten die Carbonsäuregruppen-haltigen Polymere wenigstens ein Monomer m2) einpolymerisiert, das ausgewählt ist unter Verbindungen der allgemeinen Formel (VI)

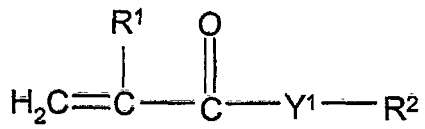

(VI)

worin

| | |
|---|---|
| $R^1$ | für Wasserstoff oder $C_1$-$C_8$-Alkyl steht, |
| $Y^1$ | für O, NH oder $NR^3$ steht, und |
| $R^2$ und $R^3$ | unabhängig voneinander für $C_1$-$C_{30}$-Alkyl oder $C_5$-$C_8$-Cycloalkyl stehen, wobei die Alkylgruppen durch bis zu vier nicht benachbarte Heteroatome öder Heteroatom-haltige Gruppen, die ausgewählt sind unter O, S und NH unterbrochen sein können. |

Bevorzugt steht $R^1$ in der Formel VI für Wasserstoff oder $C_1$-$C_4$-Alkyl, insbesondere Wasserstoff, Methyl oder Ethyl.

Bevorzugt steht $R^2$ in der Formel VI für $C_1$-$C_8$-Alkyl, bevorzugt Methyl, Ethyl, n-Butyl, Isobutyl, tert.-Butyl oder eine Gruppe der Formel $-CH_2-CH_2-NH-C(CH_3)_3$.

Wenn $R^3$ für Alkyl steht, dann vorzugsweise für $C_1$-$C_4$-Alkyl, wie Methyl, Ethyl, n-Propyl, n-Butyl, Isobutyl und tert.-Butyl.

Geeignete Monomere m2) sind Methyl(meth)acrylat, Methylethacrylat, Ethyl(meth)acrylat, Ethylethacrylat, tert.-Butyl(meth)acrylat, tert.-Butylethacrylat, n-Octyl(meth)acrylat, 1,1,3,3-Tetramethylbutyl(meth)acrylat, Ethylhexyl (meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl (meth)acrylat und Mischungen davon.

Geeignete Monomere m2) sind weiterhin Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N,N-Dimethyl(meth)acrylamid, N,N-Diethyl(meth)acrylamid Piperidinyl(meth)acrylamid und Morpholinyl(meth)acrylamid, N-(n-Octyl)(meth)acrylamid, N-(1,1,3,3-Tetramethylbutyl)(meth)acrylamid, N-Ethylhexyl(meth)acrylamid, N-(n-Nonyl)(meth) acrylamid, N-(n-Decyl)(meth)acrylamid, N-(n-Undecyl)(meth)acrylamid, N-Tridecyl(meth)acrylamid, N-Myristy/(meth)acrylamid, N-Pentadecyl(meth)acrylamid, N-Palmityl(meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl (meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid und N-Lauryl(meth)acrylamid.

Desweiteren bevorzugt enthalten die Carbonsäuregruppen-haltigen Polymere wenigstens ein Monomer m3) einpolymerisiert, das ausgewählt ist unter Verbindungen der allgemeinen Formel VII

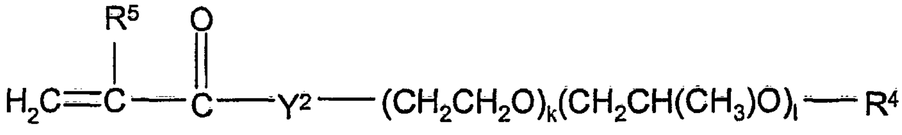

(VII)

worin
die Reihenfolge der Alkylenoxideinheiten beliebig ist,
k und l unabhängig voneinander für eine ganze Zahl von 0 bis 1000 stehen, wobei die Summe aus k und l mindestens 5 beträgt,

$R^4$ für Wasserstoff, $C_1$-$C_{30}$-Alkyl oder $C_5$-$C_8$-Cycloalkyl steht,

$R^5$ für Wasserstoff oder $C_1$-$C_8$-Alkyl steht,

$Y^2$ für O oder $NR^6$ steht, wobei $R^6$ für Wasserstoff, $C_1$-$C_{30}$-Alkyl oder $C_5$-$C_8$-Cycloalkyl steht.

Bevorzugt steht in der Formel VII k für eine ganze Zahl von 1 bis 500, insbesondere 3 bis 250. Bevorzugt steht l für eine ganze Zahl von 0 bis 100.

Bevorzugt steht $R^5$ für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht $R^4$ in der Formel VII für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl.

Vorzugsweise steht $Y^2$ in der Formel VII für O oder NH.

Geeignete Polyetheracrylate VII) sind z.B. die Polykondensationsprodukte der zuvor genannten $\alpha,\beta$-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol $R^4$-OH hergestellt werden. Die Alkylenoxide können einzeln, alternierend nacheinander oder als Mischung eingesetzt werden. Die Polyetheracrylate VII) können allein oder in Mischungen zur Herstellung der erfindungsgemäß eingesetzten Polymere verwendet werden. Geeignete Polyetheracrylate II) sind auch Urethan(meth)acrylate mit Alkylenoxidgruppen. Derartige Verbindungen sind in der DE 198 38 851 (Komponente e2)) beschrieben, worauf hier in vollem Umfang Bezug genommen wird.

Als Carbonsäuregruppen-haltige Polymere bevorzugte anionische Polymere sind beispielsweise Homo- und Copolymerisate von Acrylsäure und Methacrylsäure und deren Salze. Dazu zählen auch vernetzte Polymere der Acrylsäure, wie sie unter dem INCI-Namen Carbomer erhältlich sind. Derartige vernetzte Homopolymere der Acrylsäure sind beispielsweise kommerziell unter dem Namen Carbopol® von der Firma Noveon erhältlich. Bevorzugt sind auch hydrophob modifizierte vernetzte PolyacrylatPolymere, wie Carbopol® Ultrez 21 von der Firma Noveon.

Polyelektrolyt-Komplexe auf Basis von Homo- und Copolymerisaten von Acrylsäure und Methacrylsäure eignen sich in vorteilhafter Weise zur Formulierung als Gele, beispielsweise für Festigergele, sowie zur Formulierung von Schäumen.

Weitere Beispiele für geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem $C_8$-$C_{30}$-Alkylrest terminiert ist. Dazu zählen z.B. Acrylat/Beheneth-25-methacrylat-Copolymere, die unter der Bezeichnung Aculyn® von der Firma Rohm und Haas erhältlich sind. Besonders geeignete Polymere sind weiterhin Copolymere aus t-Butylacrylat, Ethylacrylat, Methacrylsäure (z.B. Luvimer® 100P, Luvimer® Pro55), Copolymere aus Ethylacrylat und Methacrylsäure (z.B. Luviumer® MAE), Copolymere aus N-tert.-Butylacrylamid, Ethylacrylat, Acrylsäure (Ultra-hold® 8, Ultrahold® Strong), Copolymere aus Vinylacetat, Crotonsäure und gegebenenfalls weitere Vinylester (z.B. Luviset® Marken), Maleinsäureanhydridcopolymere, gegebenenfalls mit Alkohol

umgesetzt, anionische Polysiloxane, z.B. carboxyfunktionelle, t-Butylacrylat, Methacrylsäure (z.B. Luviskol® VBM), Copolymere von Acrylsäure und Methacrylsäure mit hydrophoben Monomeren, wie z.B. $C_4$-$C_{30}$-Alkylester der Meth(acrylsäure), $C_4$-$C_{30}$-Alkylvinylester, $C_4$-$C_{30}$-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und VinylpyrrolidonNinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der geeigneten anionischen Polymere beispielhaft Balance® CR (National Starch; Acrylate Copolymer), Balance® 0/55 (National Starch; Acrylate Copolymer), Balance® 47 (National Starch; Octylacrylamid/- Acrylate/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylate Copolymer), Allianz® LT-120 (ISP / Rohm & Haas; Acrylat/C1-2 Succinat/Hydroxyacrylat Copolymer), Aquarez® HS (Eastman; Polyester-1), Diaformer® Z-400 (Clariant; Methacryloylethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/ Maleinsäure in Ethanol), Amphomer® HC (National Starch; Acrylat/ Octylacrylamid-Copolymer), Amphomer® 28-4910 (National Starch; Octyl-acrylamid/Acrylat/ Butylaminoethylmethacrylat-Copolymer), Advantage® HC 37 (ISP; Terpolymer aus Vinylcaprolactam/Vinylpyrrolidon/ Dimethylaminoethylmethacrylat), Advantage® LC55 und LC80 oder LC A und LC E, Advantage® Plus (ISP; VA/Butyl Maleate/Isobornyl Acrylate Copolymer), Aculyne® 258 (Rohm & Haas; Acrylat/ Hydroxyesteracrylat-Copolymer), Luviset® P.U.R. (BASF, Polyurethane-1), Luviflex® Silk (BASF), Eastman® AQ 48 (Eastman), Styleze® CC-10 (ISP; VP/ DMAPA Acrylates Copolymer), Styleze® 2000 (ISP; VP/AcrylateslLauryl Methacrylate Copolymer), DynamX® (National Starch; Polyurethane-14 AMP-Acrylates Copolymer), Resyn XP® (National Starch; Acrylates/Octylacrylamide Copolymer), Fixomer® A-30 (Ondeo Nalco; Polymethacrylsäure (und) Acrylamidomethylpropansulfonsäure), Fixate® G-100 (Noveon; AMP-Acrylates/Allyl Methacrylate Copolymer).

Geeignete Carbonsäuregruppen-haltige Polymere sind auch die in der US 3,405,084 beschriebenen Terpolymere aus Vinylpyrrolidon, $C_1$-$C_{10}$-Alkyl- Cycloalkyl- und Aryl(meth)acrylaten und Acrylsäure. Geeignete Carbonsäuregruppen-haltige Polymere sind weiterhin die in der EP-A-0 257 444 und EP-A-0 480 280 beschriebenen Terpolymere aus Vinylpyrrolidon, tert.-Butyl(meth)acrylat und (Meth)acrylsäure. Geeignete Carbonsäuregruppen-haltige Polymere sind weiterhin die in der DE-A-42 23 066 beschriebenen Copolymere, die wenigstens einen (Meth)acrylsäureester, (Meth)acrylsäure sowie N-Vinylpyrrolidon und/oder N-Vinylcaprolactam einpolymerisiert enthalten. Auf die Offenbarung dieser Dokumente wird hier Bezug genommen.

Die Herstellung der zuvor genannten Carbonsäuregruppen-haltigen Polymere erfolgt nach bekannten Verfahren, zum Beispiel der Lösungs-, Fällungs-, Suspensions- oder Emulsionspolymerisation, wie zuvor für die Copolymere A) beschrieben.

Geeignete Carbonsäuregruppen-haltige Polymere sind weiterhin Carbonsäuregruppen-haltige Polyurethane.

Die EP-A-636361 offenbart geeignete Blockcopolymere mit Polysiloxanblöcken und Polyurethan-/Polyharnstoffblöcken, die Carbonsäure- und/oder Sulfonsäuregruppen aufweisen. Geeignete siliconhaltige Polyurethane sind auch in der WO 97/25021 und der EP-A-751 162 beschrieben.

Geeignete Polyurethane sind auch in der DE-A-42 25 045 beschrieben, worauf hier in vollem Umfang Bezug genommen wird.

Die Säuregruppen der Carbonsäuregruppen-haltigen Polymere können teilweise oder vollständig neutralisiert sein. Dann liegt wenigstens ein Teil der Säuregruppen in deprotonierter Form vor, wobei die Gegenionen vorzugsweise ausgewählt sind unter Alkalimetallionen, wie $Na^+$, $K^+$, Ammoniumionen und deren organischen Derivaten etc.

Die erfindungsgemäßen Zubereitungen können auch im Bereich der Pharmazie eingesetzt werden.

Die Formulierungsgrundlage erfindungsgemäßer pharmazeutischer Zubereitungen enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z.B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Zubereitungen geeignet als Hilfsmittel in der Pharmazie, bevorzugt in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Zubereitungen um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und - cremes.

Nach einer weiteren bevorzugten Ausführungsform handelt es sich bei den erfindungsgemäßen Zubereitungen um kosmetische Mittel zur Pflege und zum Schutz der Haut, Nagelpflegemittel oder Zubereitungen für die dekorative Kosmetik.

Geeignete hautkosmetische Mittel sind z.B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte. Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Zubereitungen zeigen vorteilhafte Wirkungen. Die Zubereitungen können unter anderem zur Feuchthaltung und Konditionierung der Haut und zur Verbesserung des Hautgefühls beitragen. Die Zubereitungen können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Erfindungsgemäße hautkosmetische und dermatologische Zubereitungen enthalten vorzugsweise wenigstens Copolymer A) in einem Anteil von etwa 0,001 bis 30 Gew.- %, vorzugsweise 0,01 bis 20 Gew.-%, ganz besonders bevorzugt 0,1 bis 12 Gew.-%, bezogen auf das Gesamtgewicht der Zubereitung.

Besonders Lichtschutzmittel auf Basis der Copolymere A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Zubereitungen in einer zur Hautpflege geeigneten Form, wie z.B. als Creme, Schaum, Gel, Stift, Mousse, Milch, Spray (Pumpspray oder treibmittelhaltiger Spray) oder Lotion appliziert werden.

Die erfindungsgemäßen hautkosmetischen Zubereitungen können neben den Copolymeren A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von $C_6$-$C_{30}$-Fettsäuren, Wachsester, wie z.B. Jojobaöl, Fettalkohole, Vaseline, hydriertes Lanolin und acetyliertes Lanolin sowie Mischungen davon.

Die erfindungsgemäßen Zubereitungen können auch herkömmliche Polymeren enthalten, falls spezielle Eigenschaften eingestellt werden sollen.

Zur Einstellung bestimmter Eigenschaften wie z.B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Zubereitungen in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Copolymer A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann eine erfindungsgemäße Zubereitung eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesämöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Copolymeren A) können auch Wachse verwendet werden, wie z.B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Zubereitungen um ein Duschgel, eine Shampoo-Formulierung oder ein Badepräparat.

Solche erfindungsgemäßen Formulierungen enthalten wenigstens ein Copolymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z.B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z.B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder-dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z.B. quaternäre Ammoniumverbindungen, beispielsweise Cetyltrimethylammoniumchlorid.

Weiterhin können die Duschgel-/Shampoo-Formulierungen Verdicker, wie z.B. Kochsalz, PEG-55, Propyleneglykol-Oleat, PEG-120-Methylglucosedioleat und andere, sowie Konservierungsmittel, weitere Wirk- und Hilfsstoffe und

Wasser enthalten.

Nach einer weiteren bevorzugten Ausführungsform handelt es sich bei den erfindungsgemäßen Zubereitungen um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform

A) 0,05 bis 20 Gew.-% wenigstens eines Copolymeren A)
B) 20 bis 99,95 Gew.-% Wasser und/oder Alkohol,
C) 0 bis 50 Gew.-% wenigstens eines Treibgases,
D) 0 bis 5 Gew.-% wenigstens eines Emulgators,
E) 0 bis 3 Gew.-% wenigstens eines Verdickers, sowie
F) bis zu 25 Gew.-% weitere Bestandteile

wobei der pH-Wert der Formulierung wird einen Wert im Bereich von pH 4 bis pH 6 aufweist.

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Hairstyling-Zubereitungen, insbesondere Haarsprays (Aerosolsprays und Pumpsprays ohne Treibgas) und Haarschäume (Aerosolschäume und Pumpschäume ohne Reibgas).

In einer bevorzugten Ausführungsform enthalten Spray-Zubereitungen

A) 0,1 bis 3 Gew.-% wenigstens eines Copolymeren A),
B) 0,1 bis 3 Gew.-% wenigstens eines weiteren Polymers,
C) 20 bis 99,9 Gew.-% Wasser und/oder Alkohol,
D) 0 bis 70 Gew.-% wenigstens eines Treibmittel,
E) 0 bis 20 Gew.-% weitere Bestandteile.

wobei der pH-Wert der Zubereitung einen Wert im Bereich von pH 4 bis pH 6 aufweist.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

A) 0,1 bis 10 Gew.-% wenigstens eines Copolymeren A),
B) 55 bis 99,8 Gew.-% Wasser und/oder Alkohol,
C) 5 bis 20 Gew.-% eines Treibmittel,
D) 0,1 bis 5 Gew.-% eines Emulgators,
E) 0 bis 10 Gew.-% weitere Bestandteile,

wobei der pH-Wert der Zubereitung einen Wert im Bereich von pH 4 bis pH 6 aufweist.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cetethe, z.B. Cetheth-1, Polyethylenglycolcetylether; Ceteareth, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

A) 0,1 bis 10 Gew.-% wenigstens eines Copolymeren A),
B) 80 bis 99,85 Gew.-% Wasser und/oder Alkohol,
C) 0 bis 3 Gew.-%, bevorzugt 0,05 bis 2 Gew.-%, eines Gelbildners,
D) 0 bis 20 Gew.-% weitere Bestandteile,

wobei der pH-Wert der Zubereitung einen Wert im Bereich von pH 4 bis pH 6 aufweist.

Der Einsatz von Gelbildnern kann von Vorteil sein, um spezielle rheologische oder andere anwendungstechnische Eigenschaften der Gele einzustellen. Als Gelbildner können alle in der Kosmetik üblichen Gelbildner eingesetzt werden.

Hierzu zählen beispielsweise Cellulosederivate, z.B. Hydroxypropylcellulose, Hydroxyethylcellulose, kationisch modifizierte Cellulosen, Polysaccharide, z.B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Die erfindungsgemäßen Zubereitungen können in kosmetischen Zubereitungen als Konditioniermittel eingesetzt werden.

Die erfindungsgemäßen Zubereitungen können bevorzugt in Shampooformutierungen als Festigungs- und/oder Konditioniermittel eingesetzt werden. Bevorzugte Shampooformulierungen enthalten

A) 0,05 bis 10 Gew.-% wenigstens eines Copolymeren A),
B) 25 bis 94,95 Gew.-% Wasser,
C) 5 bis 50 Gew.-% Tenside,
D) 0 bis 5 Gew.-% eines weiteren Konditioniermittels,
E) 0 bis 10 Gew.-% weitere kosmetische Bestandteile,

wobei der pH-Wert der Zubereitung einen Wert im Bereich von pH 4 bis pH 6 aufweist.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Copolymeren A) eingesetzt werden. Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat® FC, Luviquat® HM, Luviquat® MS, Luviquat® Care, Luviquat® UltraCare), Copolymere aus N-Vinylpyrrolidon/Dimethylaminoethylmethacrylat, quaternisiert mit Diethylsulfat (Luviquat® PQ 11), Copolymere aus N-Vinylcaprolactam/ N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die Erfindung wird anhand der folgenden nicht einschränkenden Beispiele näher erläutert.

Beispiele

Die Monomeren sind als Gewicht-% angegeben.

1. Herstellung von Copolymerisaten (radikalische Polymerisation in Lösung)

Beispiel S1: Copolymer aus N-Vinylpyrrolidon (VP) / N-Vinylimidazol (VI) / Methacrylamid (MAM) (55/10/35)

| | |
|---|---|
| Vorlage: | Monomerengemisch aus |
| 121.5 g | voll entsalztes (=VE) Wasser |
| 17.5 g | Methacrylamid-Lösung (15 Gew.-%ig in Wasser) |
| 0.75 g | N-Vinylimidazol |
| 4.12 g | N-Vinylpyrrolidon |
| 0.03 g | Wako® V 50 [2,2'-Azobis(2-amidinopropan)-dihydrochlorid] |

| | |
|---|---|
| Zulauf 1: | Monomerengemisch aus: |
| 78.5 g | N-Vinylpyrrolidon |
| 14.2 g | N-Vinylimidazol |
| 332 g | Methacrylamid -Lösung (15 Gew.-%ig in Wasser) |

| | |
|---|---|
| Zulauf 2: | Initiatorlösung aus: |
| 0.6 g | Wako® V 50 |
| 20.97 g | VE-Wasser |

| | |
|---|---|
| Zulauf 3: | Initiatorlösung aus: |
| 0.45 g | Wako® V 50 |
| 10.5 g | VE-Wasser |

In einem 1l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Bei Erreichen der Temperatur von 70°C wurden Zulauf 1 in drei und Zulauf 2 in vier Stunden zudosiert. Anschließend wurde zwei Stunden nachpolymerisiert.

Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S2: Copolymer aus N-Vinylpyrrolidon (VP)/N-Vinylimidazol (VI)/ Methacrylamid (MAM) / N-Vinylimidazol-Methosulphat (QVI) (60/5/30/5)

| | |
|---|---|
| Vorlage: | Monomerengemisch aus |
| 164 g | VE-Wasser |
| 14.4 g | Methacrylamid-Lösung (15 Gew.-%ig in Wasser) |
| 0.36 g | N-Vinylimidazol |
| 4.32 g | N-Vinylpyrrolidon |
| 0.8 g | N-Vinylimidazol-Methosulphat-Lösung (45 Gew.-%ig in Wasser) |
| 0.04 g | Wako® V 50 [2,2'-Azobis(2-amidinopropan)-dihydrochlorid] |

| | |
|---|---|
| Zulauf 1: | Monomerengemisch aus: |
| 86.0 g | N-Vinylpyrrolidon |
| 7.1 g | N-Vinylimidazol |
| 285.0 g | Methacrylamid -Lösung (15 Gew.-%ig in Wasser) |
| 16.0 g | N-Vinylimidazol-Methosulphat-Lösung (45 Gew.-%ig in Wasser) |

| | |
|---|---|
| Zulauf 2: | Initiatorlösung aus: |
| 0.85 g | Wako® V 50 |

| | |
|---|---|
| 20.97 g | VE-Wasser |

| | |
|---|---|
| Zulauf 3: | Initiatorlösung aus: |
| 0.45 g | Wako® V 50 |
| 10.49 g | VE-Wasser |

In einem 1l-Pilotrührwerk mit Dosierung, Rückflusskühler, Innenthermometer und 3 separaten Zulaufvorrichtungen wurde die Vorlage unter Stickstoffatmosphäre und Rühren auf 65°C erwärmt. Bei Erreichen der Temperatur von 65°C wurden Zulauf 1 in drei und Zulauf 2 in vier Stunden zudosiert. Anschließend wurde zwei Stunden nachpolymerisiert.

Bei 65°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S3: Copolymer aus N-Vinylpyrrolidon (VP) / N-Vinylimidazol (VI) / Methacrylamid (MAM) / N-Vinylimidazol-Methosulphat (QVI) (55/5/35/5)

| | |
|---|---|
| Vorlage: | Monomerengemisch aus |
| 121.5 g | VE-Wasser |
| 17. 5 g | Methacrylamid-Lösung (15 Gew.-%ig in Wasser) |
| 0.37 g | N-Vinylimidazol |
| 4.1 g | N-Vinylpyrrolidon |
| 0.82 g | N-Vinylimidazol-Methosulphat-Lösung (45 Gew.-%ig in Wasser) |
| 0.04 g | Wako® V 50 [2,2'-Azobis(2-amidinopropan)-dihydrochlorid] |

| | |
|---|---|
| Zulauf 1: | Monomerengemisch aus: |
| 78 g | N-Vinylpyrrolidon |
| 7.1 g | N-Vinylimidazol |
| 332.5 g | Methacrylamid -Lösung (15 Gew.-%ig in Wasser) |
| 15.9 g | N-Vinylimidazol-Methosulphat-Lösung (45 Gew.-%ig in Wasser) |

| | |
|---|---|
| Zulauf 2: | Initiatorlösung aus: |
| 0.70 g | Wako® V 50 |
| 20.97 g | VE-Wasser |

| | |
|---|---|
| Zulauf 3: | Initiatorlösung aus: |
| 0.45 g | Wako® V 50 |
| 10.49 g | VE-Wasser |

In einem 1 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Zulauf 1 wurde durch Zugabe von Milchsäure auf einen pH Wert von 5.5 eingestellt. Bei Erreichen der Temperatur von 70°C wurden Zulauf 1 in drei und Zulauf 2 in vier Stunden zudosiert. Anschließend wurde zwei Stunden nachpolymerisiert.

Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S4: Copolymer aus N-Vinylpyrrolidon (VP) / N-Vinylimidazol (VI) / Methacrylamid (MAM) / N-Vinylimidazol-Methosulphat (QVI) (55/5/35/5)

| | |
|---|---|
| Vorlage: | Monomerengemisch aus |
| 121.5 g | VE-Wasser |
| 17.5 g | Methacrylamid-Lösung (15 Gew.-%ig in Wasser) |
| 0.37 g | N-Vinylimidazol |
| 4.04 g | N-Vinylpyrrolidon |
| 0.82 g | N-Vinylimidazol-Methosulphat-Lösung (45 Gew.-%ig in Wasser) |
| 0.04 g | Wako® V 50 [2,2'-Azobis(2-amidinopropan)-dihydrochlorid] |

| | |
|---|---|
| Zulauf 1: | Monomerengemisch aus: |
| 78 g | N-Vinylpyrrolidon |
| 7.1 g | N-Vinylimidazol |
| 332.5 g | Methacrylamid -Lösung (15 Gew.-%ig in Wasser) |
| 15.9 g | N-Vinylimidazol-Methosulphat-Lösung (45 Gew.-%ig in Wasser) |

| | |
|---|---|
| Zulauf 2: | Initiatorlösung aus: |
| 0.70 g | Wako® V 50 |
| 20.97 g | VE-Wasser |

| | |
|---|---|
| Zulauf 3: | Initiatorlösung aus: |
| 0.45 g | Wako® V 50 |
| 10.49 g | VE-Wasser |

In einem 1 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Bei Erreichen der Temperatur von 70°C wurden Zulauf 1 in drei und Zulauf 2 in vier Stunden zudosiert. Anschließend wurde zwei Stunden nachpolymerisiert.
Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S5: Copolymer aus N-Vinylpyrrolidon (VP) / N-Vinylimidazol (VI) / Methacrylamid (MAM) (55/10/35)

| | |
|---|---|
| Vorlage: | Monomerengemisch aus |
| 121.5 g | VE-Wasser |
| 17.5 g | Methacrylamid-Lösung (15 Gew.-%ig in Wasser) |
| 0.75 g | N-Vinylimidazol |
| 4.10 g | N-Vinylpyrrolidon |
| 0.03 g | Wako® V 50 [2,2'-Azobis(2-amidinopropan)-dihydrochlorid] |

| | |
|---|---|
| Zulauf 1: | Monomerengemisch aus: |
| 78.0 g | N-Vinylpyrrolidon |
| 14.0 g | N-Vinylimidazol |
| 332.5 g | Methacrylamid -Lösung (15 Gew.-%ig in Wasser) |

| | |
|---|---|
| Zulauf 2: | Initiatorlösung aus: |
| 0.55 g | Wako® V 50 |
| 20.97 g | VE-Wasser |

| | |
|---|---|
| Zulauf 3: | Initiatorlösung aus: |
| 0.45 g | Wako® V 50 |
| 10.49 g | VE-Wasser |

In einem 1 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Zulauf 1 wurde durch Zugabe von Milchsäure auf einen pH Wert von 5.5 eingestellt Bei Erreichen der Temperatur von 70°C wurden Zulauf 1 in drei und Zulauf 2 in vier Stunden zudosiert. Anschließend wurde zwei Stunden nachpolymerisiert.
Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt..

Beispiel S6: Copolymer aus N-Vinylpyrrolidon (VP) / N-Vinylirrüdazöl (VI) / Methacrylamid (MAM) (55/10/35)

| | |
|---|---|
| Vorlage: | Monomerengemisch aus |
| 121 g | VE-Wasser |
| 17.65 g | Methacrylamid-Lösung (15 Gew.-%ig in Wasser) |
| 0.7 g | N-Vinylimidazol |
| 3.77 g | N-Vinylpyrrolidon |
| 0.03 g | Wako® V 50 [2,2'-Azobis(2-amidinopropan)-dihydrochlorid] |
| 0.3 g | Milchsäure |

| | |
|---|---|
| Zulauf 1: | Monomerengemisch aus: |
| 79.0 g | N-Vinylpyrrolidon |
| 14.0 g | N-Vinylimidazol |

| | |
|---|---|
| Zulauf 2: | Monomerengemisch aus: |
| 332.0 g | Methacrylamid -Lösung (15 Gew.-%ig in Wasser) |
| 4.8 g | Milchsäure |

| | |
|---|---|
| Zulauf 3: | Initiatorlösung aus: |
| 0.6 g | Wako® V 50 |
| 20.97 g | VE-Wasser |

| | |
|---|---|
| Zulauf 4: | Initiatorlösung aus |
| 0.45 g | Wako® V 50 |
| 10.5 g | VE-Wasser |

In einem 1 l-Pilotrührwerk mit Dosierung, Rückflusskühler, Innenthermometer und 4 separaten Zulaufvorrichtungen wurde die Vorlage unter Stickstoffatmosphäre und Rühren auf 70°C erwärmt. Bei Erreichen der Temperatur von 70°C wurden Zulauf 1 in drei und die Zuläufe 2 und 3 in vier Stunden zudosiert. Anschließend wurde zwei Stunden nachpolymerisiert. Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S7: Copolymer aus N-Vinylpyrrolidon (VP) / N-Vinylimidazol (VI) / Methacrylamid (MAM) / N-Vinylimidazol-Methosulphat (QVI) (55/5/35/5)

| | |
|---|---|
| Vorlage: | Monomerengemisch aus |
| 121.5 g | VE-Wasser |
| 17.5 g | Methacrylamid-Lösung (15 Gew.-%ig in Wasser) |
| 0.37 g | N-Vinylimidazol |
| 4.10 g | N-Vinylpyrrolidon |
| 0.83 g | N-Vinylimidazol-Methosulphat-Lösung (45 Gew.-%ig in Wasser) |
| 0.04 g | Wako® V 50 [2,2'-Azobis(2-amidinopropan)-dihydrochlorid] |
| Zulauf 2: | Monomerengemisch aus: |
| 79.0 g | N-Vinylpyrrolidon |
| 16.0 | N-Vinylimidazol-Methosulphat-Lösung (45 Gew.-%ig in Wasser) |
| 7.1 g | N-Vinylimidazol |

(fortgesetzt)

| | |
|---|---|
| Zulauf 1: | Monomerengemisch aus: |
| 332.0 g | Methacrylamid -Lösung (15 Gew.-%ig in Wasser) |
| Zulauf 3: | Initiatorlösung aus: |
| 0.70 g | Wako® V 50 |
| 20.97 g | VE-Wasser |
| Zulauf 4: | Initiatorlösung aus |
| 0.45 g | Wako® V 50 |
| 10.49 g | VE-Wasser |

In einem 1 l-Pilotrührwerk mit Dosierung, Rückflusskühler, Innenthermometer und 4 separaten Zulaufvorrichtungen wurde die Vorlage unter Stickstoffatmosphäre und Rühren auf 65°C erwärmt. Zulauf 2 wurde durch Zugabe von Milchsäure auf einen pH Wert von 6.0 eingestellt. Bei Erreichen der Temperatur von 65°C wurden die Zuläufe 1 und 3 in vier Stunden und Zulauf 2 drei Stunden zudosiert. Anschließend wurde zwei Stunden nachpolymerisiert. Bei 65°C wurde Zulauf 4 in 10 Minuten zudosiert und nochmals vier Stunden nachpolymerisiert. Anschließend wurde für 30 Minuten wasserdampfdestilliert und auf 40°C abgekühlt, der pH-Wert durch Zugabe von Milchsäure auf einen Wert von pH 5.5 eingestellt.

Beispiel S8: Copolymer aus N-Vinylpyrrolidon (VP) / N-Vinylimidazol (VI) / Methacrylamid (MAM) / N-Vinylimidazol-Methosulphat (QVI) (55/8/29/8)

| | |
|---|---|
| Vorlage: | Monomerengemisch aus |
| 166.0 g | VE-Wasser |
| 15.7 g | Methacrylamid-Lösung (15 Gew.-%ig in Wasser) |
| 0.65 g | N-Vinylimidazol |
| 4.45 g | N-Vinylpyrrolidon |
| 1.45 g | N-Vinylimidazol-Methosulphat-Lösung (45 Gew.-%ig in Wasser) |
| 0.04 g | Wako® V 50 [2,2'-Azobis(2-amidinopropan)-dihydrochlorid] |

| | |
|---|---|
| Zulauf 1: | Monomerengemisch aus: |
| 274.0 g | Methacrylamid -Lösung (15 Gew.-%ig in Wasser) |

| | |
|---|---|
| 78 g | N-Vinylpyrrolidon |
| 25.2 g | N-Vinylimidazol-Methosulphat-Lösung (45 Gew.-%ig in Wasser) |
| 11.3 g | N-Vinylimidazol |

| | |
|---|---|
| Zulauf 2: | Initiatorlösung aus: |
| 0.70 g | Wako® V 50 |
| 20.97 g | VE-Wasser |

| | |
|---|---|
| Zulauf 3: | Initiatorlösung aus |
| 0.45 g | Wako® V 50 |
| 10.49 g | VE-Wasser |

In einem 1 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Zulauf 1 wurde durch Zugabe von

Milchsäure auf einen pH Wert von 6.0 eingestellt. Bei Erreichen der Temperatur von 70°C wurde Zulauf 1 in drei Stunden und Zulauf 2 vier Stunden zudosiert. Anschließend wurde zwei Stunden nachpolymerisiert. Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt. Der pH-Wert wurde durch Zugabe von Milchsäure auf einen Wert von pH 5.5 eingestellt.

Beispiel S9: Copolymer aus N-Vinylpyrrolidon (VP) / N-Vinylimidazol (VI) / Methacrylamid (MAM) / N-Vinylimidazol-Methochlorid (QVI) (55/10/29/6)

| | |
|---|---|
| Vorlage: | Monomerengemisch aus |
| 166.0 g | VE-Wasser |
| 15.7 g | Methacrylamid-Lösung (15 Gew.-%ig in Wasser) |
| 0.80 g | N-Vinylimidazol |
| 4.45 g | N-Vinylpyrrolidon |
| 1.10 g | N-Vinylimidazol-Methochlorid-Lösung (45 Gew.-%ig in Wasser) |
| 0.04 g | Wako® V 50 [2,2'-Azobis(2-amidinopropan)-dihydrochlorid] |

| | |
|---|---|
| Zulauf 1: | Monomerengemisch aus: |
| 274.0 g | Methacrylamid -Lösung (15 Gew.-%ig in Wasser) |
| 78.0 g | N-Vinylpyrrolidon |
| 19.0 g | N-Vinylimidazol-Methochlorid-Lösung (45 Gew.-%ig in Wasser) |
| 14.2 g | N-Vinylimidazol |

| | |
|---|---|
| Zulauf 2: | Initiatorlösung aus: |
| 0.70g | Wako® V 50 |
| 20.97 g | VE-Wasser |

| | |
|---|---|
| Zulauf 3: | Initiatorlösung aus |
| 0.45 g | Wako® V 50 |
| 10.49 g | VE-Wasser |

In einem 1 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Zulauf 1 wurde durch Zugabe von Milchsäure auf einen pH Wert von 6.0 eingestellt. Bei Erreichen der Temperatur von 70°C wurde Zulauf 1 in drei Stunden und Zulauf 2 vier Stunden zudosiert. Anschließend wurde zwei Stunden nachpolymerisiert. Bei 70°C wurde Zulauf 3 in 10 Minuten zudosiert und nochmals vier Stunden nachpolymerisiert. Anschließend wurde für 30 Minuten wasserdampfdestilliert und auf 40°C abgekühlt.

Beispiel S10: Copolymer aus N-Vinylpyrrolidon (VP) / N-Vinylimidazol (VI) / Methacrylamid (MAM) / N-Vinylimidazol-Methosulphat (QVI) (55/10/29/6)

| | |
|---|---|
| Vorlage: | Monomerengemisch aus |
| 324.0 g | VE-Wasser |
| 31.8 g | Methacrylamid-Lösung (15 Gew.-%ig in Wasser) |
| 1.6 g | N-Vinylimidazol |
| 9.0 g | N-Vinylpyrrolidon |
| 2.2 g | N-Vinylimidazol-Methosulphat-Lösung (45 Gew.-%ig in Wasser) |
| 0.08 g | Wako® V 50 [2,2'-Azobis(2-amidinopropan)-dihydrochlorid] |

| | |
|---|---|
| Zulauf 1: | Monomerengemisch aus: |
| 548.0 g | Methacrylamid -Lösung (15 Gew.-%ig in Wasser) |
| 156 g | N-Vinylpyrrolidon |
| 37.8 g | N-Vinylimidazol-Methosulphat-Lösung (45 Gew.-%ig in Wasser) |
| 28.5 g | N-Vinylimidazol |

| | |
|---|---|
| Zulauf 2: | Initiatorlösung aus: |
| 1.4 g | Wako® V 50 |
| 41.9 g | VE-Wasser |

| | |
|---|---|
| Zulauf 3: | Lösung aus |
| 1.28 g | tert.-Butylhydroperoxid 70%ig |
| 20 g | VE-Wasser |

| | |
|---|---|
| Zulauf 4: | Lösung aus |
| 0.76 g | Natriumdisulfit |
| 11 g | VE-Wasser |

In einem 2 l-Pilotrührwerk mit Dosierung, Rückflusskühler, Innenthermometer und 3 separaten Zulaufvorrichtungen wurde die Vorlage unter Stickstoffatmosphäre und Rühren auf 70°C erwärmt. Zulauf 1 wurde durch Zugabe von Phosphorsäure auf einen pH Wert von 6.5 eingestellt. Bei Erreichen der Temperatur von 70°C wurde Zulauf 1 in drei Stunden und Zulauf 2 vier Stunden zudosiert. Anschließend wurde zwei Stunden nachpolymerisiert. Die Temperatur wurde auf 75°C erhöht. Bei 75°C wurde Zulauf 3 batch zudosiert. Nach 10 Min wurde Zulauf 4 in 2 Portionen innerhalb von 5 Minuten zugegeben und 3 Stunden nachpolymerisiert. Anschließend wurde für 30 Minuten wasserdampfdestilliert und auf 40°C abgekühlt.

Anwendungsbeispiele

Die laut den Synthesebeispielen S1 bis S10 synthetisierten Copolymere wurden entsprechend folgender Zusammensetzung als Schaumformulierungen (Beispiele SF1 bis SF16) präpariert.

| | |
|---|---|
| SF1- SF16: | Schaumformulierungen: |
| 2,00 g | Luviquat® Mono LS |
| 0,20 g | Parfümöl |
| 2,00 g | WS* Polymer |
| 0,10 g | Euxyl® K 100 |
| q.s. | Phenonip® |
| ad 100 g | Wasser dem. |
| 10,00 g | Propan/Butan 3,5 bar |

Herstellung: Die Komponenten wurden eingewogen und bei Raumtemperatur unter Rühren langsam homogenisiert. Danach wurde die Zubereitung in einen geeigneten Behälter abgefüllt und das Treibgas zugesetzt.
**[0282]** * WS: Wirkstoff Polymer steht für die Masse Polymer in g in 100g der Gesamtformulierung
SF2, SF4, SF7, SF9, SF14: Der pH-Wert der Schaumformulierungen auf Basis der Polymere gemäß der Angaben in Tabelle 2 wurde mit Milchsäure auf den in Tabelle 2 angegebenen pH-Wert eingestellt.
SF12 Schaumformulierung, bei der pH-Wert der wässrigen Zubereitung nach abgeschlossener Polymerisation aber vor der Formulierung als Schaum eingestellt wurde.
SF16, SF18: der pH-Wert der Schaumformulierung auf Basis des Polymers gemäß der Angabe in Tabelle 2

wurde mit Phosphorsäure auf den in Tabelle 2 angegebenen pH-Wert eingestellt.

SF1, SF3, SF5, SF6, SF8, SF10, SF13 und SF15 sind Schaumformulierungen, bei denen der pH-Wert der wässrigen Zubereitung nach abgeschlossener Polymerisation nicht eingestellt wurde (Vergleichsbeispiele).

Bestimmung des K-Wertes

Die K-Werte werden nach Fikentscher, Cellulosechemie, Bd. 13, S. 58 bis 64 (1932) bei 25°C in wässrig/ethanolischer oder ethanolischer Lösung gemessen und stellen ein Maß für das Molgewicht dar. Die wässrig/ethanolische oder ethanolische Lösung der Polymerisate enthält 1g Polymerisat in 100 ml Lösung. Für den Fall, dass die Polymerisate in Form von wässrigen Dispersionen vorliegen, werden in Abhängigkeit vom Polymergehalt der Dispersion entsprechende Mengen der Dispersion mit Ethanol auf 100 ml aufgefüllt, so dass die Konzentration von 1g Polymerisat in 100 ml Lösung entstehen. Die Messung des K-Wertes erfolgt in einer Micro-Ubbelohde-Kapillare Typ M Ic der Fa. Schott.

Berechnung des K-Wertes mit Mischungskorrektur für Wasser in Ethanol

Die unten aufgeführten Faktoren in der Gleichung der Mischungskorrektur beziehen sich ausschließlich auf diesen Kapillartyp bei einer Messtemperatur von 25°C.

Berechnung K-Wert:

$$K = k * 1000; z = \eta_{rel}$$

$$k = \frac{(1.5\log z - 1)c \pm \sqrt{[(1.5\log z - 1)^2 c^2 + 4(75c + 1.5c^2)(\log z)]}}{2(75c + 1.5c^2)}$$

Relative Viskosität:

$$\eta_{rel} = (t_{Lsg} - HC_{Lsg}) / (t_{LM} - HC_{LM})$$

Berechnung der Mischungskorrektur:

Mischungen von Wasser in Ethanol zeigen nichtproportionale Veränderungen der Viskosität des Lösemittelgemischs in Bezug auf den Anteil von Wasser.

Auf Grund der Beschaffenheit der Probe (wässrige Dispersion eines Polymers)wird in die ethanolische Probelösung durch die Probeneinwaage Wasser eingebracht. Diese Wassermenge wird durch die Mischungskorrektur in die Laufzeit des Lösemittels eingerechnet, so dass die relative Viskosität entsprechend der Zumischung von Wasser korrigiert wird.

Laufzeit Lösemittelmischung:

$$t_{LM} = t_o + t_M$$

Laufzeitkorrektur:

$$t_M = - 7{,}486100e\text{-}5 * c_W^4 + 3{,}785884\,E\text{-}3 * c_W^3$$

$$- 8{,}063441E\text{-}2 * c_W^2 + 1{,}999207 * c_W + 2{,}959258E\text{-}2$$

Lösemittelanteil Wasser:

$$c_W = c / FG / 100 * (1 - FG / 100)$$

| | |
|---|---|
| c | Konzentration der Meßlösung [g/100ml] |
| $c_w$ | Konzentration an Wasser in der Meßlösung [g/100ml] |
| FG | Feststoffgehalt der Probe [g/100g] |
| $HC_{LM}$ | Hagenbach-Korrektur des Lösemittels [-s] |
| $HC_{Lsg}$ | Hagenbach-Korrektur der Meßlösung [-s] |
| $t_{LM}$ | Durchlaufzeit des Lösemittels mischungskorrigiert [s] |
| $t_{Lsg}$ | Durchlaufzeit der Messlösung, gemessen [s] |
| $t_0$ | Durchlaufzeit des Lösemittels, gemessen [s] |
| $t_M$ | Laufzeitkorrektur für die Lösemittelmischung, berechnet [s] |
| z | $\eta_{rel}$ in der Fikentscher-Gleichung (K-Wert-Berechnung) |

Curl Retention:

Die feuchte Haarsträhne wird zwischen Filterpapier ausgedrückt, dreimal in die Polymerlösung (Formulierung ohne Treibgas) eingetaucht, zwischendurch mit den Fingern abgestreift und wieder zwischen Filterpapier abgedrückt. Danach wird das Haar um einen Teflonstab gewickelt und mit Filterpapier und Gummiring befestigt. Anschließend werden die Haarsträhnen ca. 90 min bei 70 bis 80°C im Wärmeschrank getrocknet.

Nach Abkühlen auf Raumtemperatur werden die Locken unter Erhalt der Form abgestreift und an einem eigens hierfür angefertigtem Gestell aufgehängt und die Lockenlänge ($L_0$) an der angebrachten Skala in cm gemessen.

Für die Bestimmung eines Curl Retention Wertes sind 5 Haarlocken zu verwenden. Die Locken werden hängend in eine Klimakammer mit 20°C und 75 % bzw. 90% rel. Luftfeuchte gestellt. Nach 5 Stunden wird die Länge (Lt) abgelesen.

Die Curl Retention errechnet sich wie folgt:

$$\text{Curl Retention in \%} = \frac{L - L_t}{L - L_0} * 100$$

| | |
|---|---|
| L | = Länge der Haare (15,5 cm) |
| $L_0$ | = Länge der Haarlocke nach dem Trocknen |
| $L_t$ | = Länge der Haarlocke nach Klimabehandlung |

Als Curl Retention wird der Mittelwert aus den 5 Einzelmessungen nach 5 h bei 20°C und 75 % bzw. 90 % rel. Feuchte angegeben.

Biegefestigkeit:

Die trockenen, gewogenen Strähnen werden dreimal in VE-Wasser getaucht, abgestreift, zwischen Filterpapier abgedrückt und gewogen. Anschließend werden die Strähnen dreimal in die zu prüfende Polymerlösung (Formulierung ohne Treibgas) getaucht, beim Herausziehen mit den Fingern abgestreift und ebenfalls zwischen Filterpapier abgedrückt

und gewogen. Danach wird die Strähne von Hand so geformt, daß der Querschnitt möglichst rund wird. Die Strähne wird zum Trocknen freihängend mit einer Klammer in einem Klimaraum (21 °C und 65 % rel. Feuchte) über Nacht aufgehängt.

Die Prüfungen werden in einem Klimaraum bei 21 °C und 65 % rel. Luftfeuchte mittels eines Zug/Druck-Prüfgerätes (Typ Easytest 86 802, Fa. Frank) durchgeführt. Die Haarsträhne wird symmetrisch auf zwei zylindrische Rollen (Durchmesser 4 mm, Abstand 90 mm) der Probenaufnahme gelegt. In der Mitte wird nun von oben mit einem abgerundeten Stempel die Strähne 40 mm durchgebogen (Brechen des Polymerfilms). Die dafür erforderliche Kraft wird mit einer Wägezelle (50 N bzw. 10 N) gemessen und in Newton angegeben. Jede Polymerlösung wird an 5 verschiedenen Haarsträhnen geprüft.

Als Ergebnis wird der Mittelwert aus den 5 Einzelmessungen verwendet.

Die Bestimmungen des K-Wertes, des pH-Wertes, der Curl-Retention sowie der Biegefestigkeit wurden für die in Tabelle 2 aufgeführten Formulierungen durchgeführt.

Tabelle 2

| SchaumFormulierung | Polymer aus Beispiel | pH | VP [Gew.-%] | MAM [Gew.-%] | VI [Gew.-%] | QVI [Gew.-%] | K-Wert | Festigung [cN] | Curl-Retention [%] |
|---|---|---|---|---|---|---|---|---|---|
| SF1* | S1 | 7.6 | 55 | 35 | 10 | - | 92 | 356 | 95 |
| SF2 | S1 | 5.4 | 55 | 35 | 10 | - | 92 | 465 | 97 |
| SF3* | S6 | 6.7 | 55 | 35 | 10 | 86.2 | 86.2 | 323 | 99 |
| SF4 | S6 | 5.5 | 55 | 35 | 10 | - | 86.2 | 378 | 99 |
| SF6* | S2 | 7.4 | 60 | 30 | 5 | 5 | 92.8 | 388 | 92 |
| SF7 | S2 | 5.6 | 60 | 30 | 5 | 5 | 92.8 | 430 | 92 |
| SF8* | S4 | 7.4 | 55 | 35 | 5 | 5 | 97.5 | 349 | 95 |
| SF9 | S4 | 5.7 | 55 | 35 | 5 | 5 | 97.5 | 427 | 95 |
| SF10* | S3 | 6.1 | 55 | 35 | 5 | 5 | 86.8 | 256 | 94 |
| SF11 | S7 | 5.6 | 55 | 35 | 5 | 5 | 103.7 | 376 | 99 |
| SF12 | S8 | 5.8 | 55 | 29 | 8 | 8 | 102.9 | 411 | 96 |
| SF13* | S9 | 6.7 | 55 | 29 | 10 | 6 | 95.9 | 480 | 94 |
| SF14 | S9 | 5.5 | 55 | 29 | 10 | 6 | 95.9 | 550 | 97 |
| SF15* | S10 | 7.1 | 55 | 29 | 10 | 6 | 99.9 | 348 | - |
| SF16 | S10 | 5.5 | 55 | 29 | 10 | 6 | 99.9 | 406 | - |
| * Vergleichsversuche | | | | | | | | | |

PF1 bis PF11) Pumpspray

| Inhaltsstoff | Gew.-% | Bezeichnung nach CTFA | Hersteller |
|---|---|---|---|
| 20 Gew.-%ige wässrige Lösung eines der Polymere aus S1 bis S10 | 10.0 | | |
| Chitosan (2 Gew.%ige wässrige Lösung, mit Milchsäure auf pH 6 eingestellt) | 50.0 | | Sigma-Aldrich |
| Wasser destilliert | 44.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden.

Herstellung: Alle Komponenten werden eingewogen, bei Raumtemperatur unter Rühren langsam homogenisiert und der pH-Wert mit Milchsäure oder Phosphorsäure auf einen Wert von pH 5 bis pH 6 eingestellt:

GF1 bis GF11) Gelfestiger 1

| Inhaltsstoff | Gew.-% | Bezeichnung CTFA | Hersteller |
|---|---|---|---|
| 20 Gew.%ige wäss-rige Lösung eines der Polymere aus S1 bis S10 | 10.0 | | |
| Glycerin | 2.0 | | |
| Natrosol® 250 HR (2 Gew.-%ige wässrige Lösung) | 50.0 | Hydroxyethylcellulose | Hercules |
| Milchsäure oder Phosphorsäure | bis pH auf Wert im Bereich von 5 bis 6 | | |
| Wasser destilliert | auf 100 | Aqua | |

Darüberhinaus können den Zubereitungen Konservierungsmittel, lösliches ethoxiliertes Silikon, Parfümöl und weitere kosmetisch übliche Inhaltsstoffe zugegeben werden.
Herstellung: Alle Komponenten werden eingewogen, bei Raumtemperatur unter Rühren langsam homogenisiert und der pH-Wert mit Mlchsäure oder Phosphorsäure auf einen Wert von pH 5 bis pH 6 eingestellt.

GF12 bis GF22) Gelfestiger 2

| Inhaltsstoff | Gew.-% | Bezeichnung CTFA | Hersteller |
|---|---|---|---|
| 20 Gew.-%ige wässrige Lösung eines der Polymere aus S1 bis S10 | 5 | | |
| Natrosol® 250 HR | 2.0 | Hydroxyethylcellulose | Hercules |
| D-Panthenol USP | 0.5 | Panthenol | BASF |
| Karion F flüssig | 1.0 | Sorbitol | |
| Milchsäure oder Phosphorsäure ( | bis pH auf Wert im Bereich von 5 bis 6 | | |
| Wasser dest. | auf 100.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden. Herstellung: Alle Komponenten werden eingewogen und bei Raumtemperatur unter Rühren langsam homogenisiert.

HS1 bis HS11) Wässrige Handpump-Sprays

| Inhaltsstoff | Gew.-% | Bezeichnung CTFA | Hersteller |
|---|---|---|---|
| 20 Gew.-%ige wässrige Lösung eines der Polymere aus S1 bis S10 | 7.5 | | |
| Luviskol ®VA64 (30%ige Wasser/ Ethanol-Lösung) | 5.0 | VPNA Copolymer | BASF |
| Glycerin | 3.0 | | |
| Milchsäure oder Phosphorsäure | bis pH auf Wert im Bereich von 5 bis 6 | | |

(fortgesetzt)

| Inhaltsstoff | Gew.-% | Bezeichnung CTFA | Hersteller |
|---|---|---|---|
| Wasser dest. | auf 100.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden. Herstellung: Alle Komponenten werden eingewogen und bei Raumtemperatur unter Rühren langsam homogenisiert.

SF19 bis SF29) Schaumfestiger 1

| Inhaltsstoff | Gew.-% | Bezeichnung CTFA | Hersteller |
|---|---|---|---|
| 20 Gew.-%ige wässrige Lösung eines der Polymere aus S1 bis S10 | 7.5 | | |
| Cremophor® A 25 | 0.2 | Ceteareth 25 | BASF |
| Comperlan® KD | 0.1 | Coamide DEA | Henkel |
| Dimethylether | 10.0 | | |
| Milchsäure oder Phosphorsäure | bis pH auf Wert im Bereich von 5 bis 6 | | |
| Wasser dest. | auf 100.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden.

Herstellung: Die Komponenten werden eingewogen und bei Raumtemperatur unter Rühren langsam homogenisiert. Danach wird die Zubereitung abgefüllt und das Treibgas wird zugesetzt.

SF30 bis SF40) Schaumfestiger 2

| Inhaltsstoff | Gew.-% | Bezeichnung CTFA | Hersteller |
|---|---|---|---|
| 20 Gew.-%ige wässrige Lösung eines der Polymere aus S1 bis S10 | 15.0 | | |
| Cremophor® A 25 | 0.2 | Ceteareth 25 | BASF |
| Comperlan® KD | 0.1 | Coamide DEA | Henkel |
| Dimethylether | 10.0 | | |
| Milchsäure oder Phosphorsäure | bis pH auf Wert im Bereich von 5 bis 6 | | |
| Wasser dest. | auf 100.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden. Herstellung: Die Komponenten werden eingewogen und bei Raumtemperatur unter Rühren langsam homogenisiert. Danach wird die Zubereitung abgefüllt und das Treibgas wird zugesetzt.

SF41 bis SF51) Schaumfestiger 3

| Inhaltsstoff | Gew.-% | Bezeichnung CTFA | Hersteller |
|---|---|---|---|
| 20 Gew.-%ige wässrige Lösung eines der Polymere aus S1 bis S10 | 15.0 | | |
| Luviquat® Mono LS | 2.00 | Cocotrimonium Methosulfate | BASF |
| D-Panthenol USP | 1.0 | Panthenol | BASF |
| Propan/Butan | 10.0 (auf 3,5 bar) | Propane/Butane | |
| Milchsäure oder Phosphorsäure | bis pH.auf Wert im Bereich von 5 bis 6 | | |
| Wasser dest. | auf 100.0 | Aqua | |

Darüberhinaus können den Zubereitungen Konservierungsmittel, lösliches ethoxiliertes Silikon, Parfümöl und weitere kosmetische übliche Inhaltsstoffe zugegeben werden.

Herstellung: Die Komponenten werden eingewogen und bei Raumtemperatur unter Rühren langsam homogenisiert. Danach wird die Zubereitung abgefüllt und das Treibgas wird zugesetzt.

CS1 bis CS11) Conditioner Shampoo 1

| Inhaltsstoff | Gew.-% | Bezeichnung CTFA | Hersteller |
|---|---|---|---|
| | | | |
| Phase A | | | |
| Texapon® NSO 28%ig | 50.0 | Sodium Laureth Sulphate | Henkel |
| Comperlan® KD | 1.0 | Coamide DEA | Henkel |
| 20 Gew.-%ige wässrige Lösung eines der Polymere aus S1 bis S10 | 4.5 | | |
| Milchsäure oder Phosphorsäure | bis pH auf Wert im Bereich von 5 | | |
| Inhaltsstoff | Gew.-% | Bezeichnung CTFA | Hersteller |
| | bis 6 | | |
| Wasser dest. | 15.5 | Aqua | |
| Parfümöl | q.s. | | |
| | | | |
| Phase B | | | |
| Wasser dest. | 27.5 | Aqua | |
| Natriumchlorid | 1.5 | Sodium Chloride | |
| Konservierungsmittel | q.s | | |

Darüberhinaus können den Zubereitungen Konservierungsmittel, lösliches ethoxiliertes Silikon, Parfümöl und weitere kosmetische übliche Inhaltsstoffe zugegeben werden.

Einwiegen und unter Rühren Phasen A und B getrennt lösen. Phase A mit Milchsäure (20 Gew.-% in Wasser) auf einen pH-Wert von 5 bis 6 einstellen und mit Wasser auf 100 Gew.- % auffüllen. Phase B wird langsam in Phase A eingerührt.

CS12 bis CS22) Conditioner Shampoo 2

| Inhaltsstoff | Gew.-% | Bezeichnung CTFA | Hersteller |
|---|---|---|---|
| | | | |
| Phase A | | | |
| Tego Betain® L 7 | 15.00 | Cocamidopropyl Betaine | Degussa |
| Amphotensid® GB 2009 | 10,00 | Disodium Cocoamphodiacetate | Zschimmer & Schwarz |
| Cremophor® PS 20 | 5,00 | Polysorbate 20 | BASF |
| Plantacare® 2000 | 5,00 | Decyl Glucoside | Cognis |
| 20 Gew.-%ige wässrige Lösung eines der Polymere aus S1 bis S10 | 0.5 bis 5.0 | | |
| Guar Hydroypropyltrimonium Chloride | 0.15 | | |
| Rewopal® LA 3 | 2,00 | Laureth-3 | Degussa |
| Milchsäure oder Phosphorsäure | bis pH auf Wert im Bereich von 5 bis 6 | | |
| Wasser demineralisiert | auf 100 | Aqua dem | |
| Parfümöl | q.s. | | |
| Inhaltsstoff | Gew.-% | Bezeichnung CTFA | Hersteller |
| Konservierungsmittel | q.s. | | |
| | | | |
| Phase B | | | |
| Stepan® PEG 6000 DS | 3.00 | PEG-150 Distearate | Stepan Co. |

Einwiegen und unter Rühren Phasen A und B getrennt lösen. Phase A mit Milchsäure (20 Gew.-% in Wasser) auf einen pH-Wert von 5 bis 6 einstellen und mit Wasser auf 100 Gew.-% auffüllen. Phase B wird langsam in Phase A eingerührt.

CS23 bis CS33) Conditioner Shampoo 3

| Inhaltsstoff | Gew.-% | Bezeichnung CTFA | Hersteller |
|---|---|---|---|
| | | | |
| Texapon® NSO 28%ig | 30.0 | Sodium Laureth Sulphate | Henkel |
| Dehyton® G | 6,00 | Sodium Cocoamphoacetate | Henkel |
| Dehyton® K | 6,00 | Cocamidopropyl Betaine | Henkel |
| Euperlan® PK 771 | 3,00 | Sodium Laureth Sulfate, Glycol Distearate, Cocamide MEA, Laureth-10 | Henkel |
| 20 Gew.-%ige wässrige Lösung eines der Polymere aus S1 bis S10 | 0.5 bis 5.0 | | |
| Luviquat® Care | 7,70 | Polyquaternium-44 | BASF |

(fortgesetzt)

| Inhaltsstoff | Gew.-% | Bezeichnung CTFA | Hersteller |
|---|---|---|---|
| Amodimethicone | 2,00 | Amodimethicone | |
| Natriumchlorid | 1,00 | Sodium Chloride | |
| Milchsäure oder Phosphorsäure | bis pH auf Wert im Bereich von 5 bis 6 | | |
| Wasser demineralisiert | auf 100 | Aqua dem | |

Darüberhinaus können den Zubereitungen Konservierungsmittel, lösliches ethoxiliertes Silikon, Parfümöl und weitere kosmetische übliche (Inhaltsstoffe zugegeben werden. Herstellung: Die Komponenten werden eingewogen, bei Raumtemperatur unter Rühren langsam homogenisiert, der pH-Wert mit Milchsäure oder Phosphorsäure auf einen Wert von pH 5 bis pH 6 eingestellt und mit Wasser auf 100 Ges:-% aüfgefüllt.

CM1 bis CM11) Conditioner Mousse 1

| Inhaltsstoff | Gew.-% | Bezeichnung CTFA | Hersteller |
|---|---|---|---|
| Luviquat® PQ 11 | 10,00 | Polyquaternium-11 | BASF |
| 20 Gew.-%ige wässrige Lösung eines der Polymere aus S1 bis S10 | 0,5-5,0 | | |
| Luviquat® Mono CP | 0,50 | Hydroxyethyl Cetyldimonium Phosphate | BASF |
| D-Panthenol® USP | 1,00 | Panthenol | BASF |
| Milchsäure oder Phosphorsäure | bis pH auf Wert im Bereich von 5 bis 6 | | |
| Wasser demineralisiert | auf 100 | Aqua dem | |
| Propan/Butan | 6,00 | Propane/Butane | |

Darüberhinaus können den Zubereitungen Konservierungsmittel, lösliches ethoxiliertes Silikon, Parfümöl und weitere kosmetische übliche Inhaltsstoffe zugegeben werden.

Herstellung: Die Komponenten werden eingewogen, bei Raumtemperatur unter Rühren langsam homogenisiert, der pH-Wert mit Milchsäure oder Phosphorsäure auf einen Wert von pH 5 bis pH 6 eingestellt und mit Wasser auf 100 Gew.-% aufgefüllt. Danach wird die Zubereitung abgefüllt und das Treibgas wird zugesetzt.

CM12 bis CM22) Conditioner Mousse 2

| Inhaltsstoff | Gew.-% | Bezeichnung CTFA | Hersteller |
|---|---|---|---|
| Polyquaternium-4 | 1,00 | | |
| 20 Gew.-%ige wässrige Lösung eines der Polymere aus S1 bis S10 | 4,0-10,00 | | |
| Luviquat® Mono CP | 0,50 | Hydroxyethyl Cetyldimonium | BASF |
| | | Phosphate | |

(fortgesetzt)

| Inhaltsstoff | Gew.-% | Bezeichnung CTFA | Hersteller |
|---|---|---|---|
| Milchsäure oder Phosphorsäure | bis pH auf Wert im Bereich von 5 bis 6 | | |
| Wasser demineralisiert | auf 100 | Aqua dem | |
| Propan/Butan | 6,00 | Propane/Butane | |

Darüberhinaus können den Zubereitungen Konservierungsmittel, lösliches ethoxiliertes Silikon, Parfümöl und weitere kosmetische übliche Inhaltsstoffe zugegeben werden. Herstellung: Die Komponenten werden eingewogen, bei Raumtemperatur unter Rühren langsam homogenisiert, der pH-Wert mit Milchsäure oder Phosphorsäure auf einen Wert von pH 5 bis pH 6 eingestellt und mit Wasser auf 100 Gew.-% aufgefüllt Danach wird die Zubereitung abgefüllt und das Treibgas wird zugesetzt.

SM1 bis SM11) Styling Mousse 1

| Inhaltsstoff | Gew.-% | Bezeichnung CTFA | Hersteller |
|---|---|---|---|
| Phase A | | | |
| Luviquat® Mono LS | 2,00 | Cocotrimonium Methosulfate | BASF |
| Phase B | | | |
| Luviflex® Soft | 6,70 | Acrylates Copolymer | BASF |
| AMP | 0,60 | Aminomethyl Propanol | Angus Chemical Company |
| 20 Gew.-%ige wässrige Lösung eines der Polymere aus S1 bis S10 | 5,0-10,0 | | |
| Dimethicone Copolyol | 0,50 | Dimethicone Copolyol | |
| Cremophor® A 25 | 0,20 | Ceteareth-25 | BASF |
| D-Panthenol® USP | 0,20 | Panthenol | BASF |
| Uvinul® P 25 | 0,10 | PEG-25 PABA | BASF |
| Natrosol® 250 HR | 0,20 | Hydroxyethylcellulose | Aqualon GmbH |
| Milchsäure oder Phosphorsäure | bis pH auf Wert im Bereich von 5 bis 6 | | |
| Wasser demineralisiert | auf 100 | Aqua dem | |
| Inhaltsstoff | Gew.-% | Bezeichnung CTFA | Hersteller |
| Phase C | | | |
| Dymel HFC 152a | 10,00 | Hydrofluorocarbon 152a | Dupont |

Herstellung: Die Komponenten für die Phasen A und B werden eingewogen, bei Raumtemperatur unter Rühren langsam homogenisiert, der pH-Wert von Phase B mit Milchsäure oder Phosphorsäure auf einen Wert von pH 5 bis pH 6 eingestellt und Phase B mit Wasser auf 100 Gew.-% aufgefüllt. Danach werden A und B gemischt, die Zubereitung abgefüllt und das Treibgas (Phase C) zugesetzt.

SM12 bis SM22) Styling Mousse 2

| Inhaltsstoffe | Gew.-% | Bezeichnung CTFA | Hersteller |
|---|---|---|---|
| Phase A | | | |
| Luviquat® Mono LS | 2,00 | Cocotrimonium Methosulfate | BASF |
| Phase B | | | |
| Luviquat® Care | 7,70 | Polyquaternium-44 | BASF |
| 20 Gew.-%ige wässrige Lösung eines der Polymere aus S1 bis S10 | 5,0-10,0 | | |
| Milchsäure oder Phosphorsäure | bis pH auf Wert im Bereich von 5 bis 6 | | |
| Wasser demineralisiert | auf 100 | Aqua dem | |
| Phase C | | | |
| Propan/Butan | 10,00 | | |

Herstellung: Die Komponenten für die Phasen A und B werden eingewogen, bei Raumtemperatur unter Rühren langsam homogenisiert, der pH-Wert von Phase B mit Milchsäure oder Phosphorsäure auf einen Wert von pH 5 bis pH 6 eingestellt und Phase B mit Wasser auf 100 Gew.-% aufgefüllt. Danach werden A und B gemischt, die Zubereitung abgefüllt und das Treibgas (Phase C) zugesetzt.

SM23 bis SM33) Styling Mousse 3

| Inhaltsstoff | Gew.-% | Bezeichnung CTFA | Hersteller |
|---|---|---|---|
| Phase A | | | |
| Luviquat® Mono LS | 2,00 | Cocotrimonium Methosulfate | BASF |
| Phase B | | | |
| Styleze® 2000 | 2,00 | VP/Acrylates/Lauryl Methacrylate Copolymer | ISP |
| AMP | 0,53 | | |
| 20 Gew.-%ige wässrige Lösung eines der Polymere aus S1 bis S10 | 0,5-5 | | |
| Cremophor® A 25 | 0,20 | Ceteareth-25 | BASF |
| D-Panthenol USP® | 0,50 | Panthenol | BASF |
| Uvinul® MS 40 | 0,05 | Benzophenone-4 | BASF |
| Dow Corning 949® Cationic Emulsion | 0,20 | Amodimethicone/ Cetrimonium Chloride/ Trideceth-12 | Dow Corning |
| Ethanol 96% | 15,00 | Alcohol | |
| Milchsäure oder Phosphorsäure | bis pH auf Wert im Bereich von 5 bis 6 | | |
| Wasser demineralisiert | auf 100 | Aqua dem | |

(fortgesetzt)

| | | | |
|---|---|---|---|
| Phase C | | | |
| Natrosol® 250 HR | 0,20 | Hydroxyethylcellulose | Aqualon GmbH |
| Phase D | | | |
| Propan/Butan | 6,00 | Propane/Butane | |

Herstellung: Die Komponenten für die Phasen A und B werden eingewogen, bei Raumtemperatur unter Rühren langsam homogenisiert, der pH-Wert von Phase B mit Milchsäure oder Phosphorsäure auf einen Wert von pH 5 bis pH 6 eingestellt und Phase B mit Wasser auf 100 Gew.-% aufgefüllt. Danach werden A und B gemischt, Phase C zugesetzt, die Zubereitung abgefüllt und das Treibgas (Phase D) zugesetzt.

SM34 bis SM44) Styling Mousse 4

| Inhaltsstoff | Gew.-% | Bezeichnung CTFA | Hersteller |
|---|---|---|---|
| Phase A | | | |
| Cetrimonium Chloride | 2,00 | Cetrimonium Chloride | |
| Phase B | | | |
| Luviquat®Hold | 7,00 | Polyquaternium-46 | BASF |
| 20 Gew.-%ige | 0,5-5 | | |
| Inhaltsstoff | Gew.-% | Bezeichnung CTFA | Hersteller |
| wässrige Lösung eines der Polymere aus S1 bis S10 | | | |
| Cremophor® A 25 | 0,20 | Ceteareth-25 | BASF |
| D-Panthenol USP® | 0,50 | Panthenol | BASF |
| Uvinul® MS 40 | 0,05 | Benzophenone-4 | BASF |
| Dow Corning 949® Cationic | 0,20 | | Dow Corning |
| Ethanol 96% | 15,00 | Alcohol | |
| Milchsäure oder Phosphorsäure | bis pH auf Wert im Bereich von 5 bis 6 | | |
| Wasser demineralisiert | auf 100 | Aqua dem | |
| Phase C | | | |
| Natrosol® 250 HR | 0,20 | Hydroxyethylcellulose | Aqualon GmbH |
| Phase D | | | |
| Propan/Butan | 6,00 | Propane/Butane | |

Herstellung: Die Komponenten für die Phasen A und B werden eingewogen, bei Raumtemperatur unter Rühren langsam homogenisiert, der pH-Wert von Phase B mit Milchsäure oder Phosphorsäure auf einen Wert von pH 5 bis pH 6 eingestellt und Phase B mit Wasser auf 100 Gew.-% aufgefüllt. Danach werden A und B gemischt, Phase C zugesetzt, die Zubereitung abgefüllt und das Treibgas (Phase D) zugesetzt.

SM45 bis SM55) Styling Mousse 5

| Inhaltsstoff | Gew.-% | Bezeichnung CTFA | Hersteller |
|---|---|---|---|
| Phase A | | | |
| Cremophor® RH 40 | q.s. | PEG-40 Hydrogenated Castor Oil | BASF |
| Wasser demineralsiert | auf 100 | Aqua dem | |
| Phase B | | | |
| Flexan® 130 | 7,00 | Sodium Polystyrene Sulfonate | National Starch |
| 20 Gew.-%ige wässrige Lösung eines der Polymere aus S1 bis S10 | 5,0-10,0 | | |
| Cetrimonium Bromide | 0,50 | Cetrimonium Bromide | |
| Milchsäure oder Phosphorsäure | bis pH auf Wert im Bereich von 5 bis 6 | | |
| Phase C | | | |
| Propan/Butan | 6,00 | Propane/Butane | |

Herstellung: Einwiegen und unter Rühren Phasen A und B getrennt lösen und mischen. Phase B mit Milchsäure oder Phosphorsäure auf einen pH-Wert von pH 5 bis pH 6 einstellen, Phase A oder B gegebenenfalls noch weitere Stoffe wie Konservierungsmittel oder Parfümöl zusetzen. Abfüllen und Treibgas (Phase C) zusetzen.

SM56 bis SM66) Styling Mousse 6

| Inhaltsstoff | Gew.-% | Bezeichnung CTFA | Hersteller |
|---|---|---|---|
| Phase A | | | |
| Cremophor® RH 40 | q.s. | PEG-40 Hydrogenated Castor Oil | BASF |
| Wasser demineralisiert | auf 100 | Aqua dem | |
| Phase B | | | |
| UCare Polymer | 0.5 | Polyquaternium-10 | Amerchol |
| 20 Gew.-%ige wässrige Lösung eines der Polymere aus | 5,0-10,0 | | |
| Inhaltsstoff | Gew.-% | Bezeichnung CTFA | Hersteller |
| S1 bis S10 | | | |
| Cetrimonium Bromide | 0,50 | | |
| Milchsäure oder Phosphorsäure | bis pH auf Wert im Bereich von 5 bis 6 | | |
| Phase C | | | |
| Propan/Butan | 6,00 | Propane/Butane | |

Herstellung: Einwiegen und unter Rühren Phasen A und B getrennt lösen und mischen. Phase B mit Milchsäure oder Phosphorsäure auf einen pH-Wert von pH 5 bis pH 6 einstellen, Phase A oder B gegebenenfalls noch weitere Stoffe wie Konservierungsmittel oder Parfümöl zusetzen. Abfüllen und Treibgas (Phase C) zusetzen.

SM67 bis SM77) Styling Mousse 7

| Inhaltsstoff | Gew.-% | Bezeichnung CTFA | Hersteller |
|---|---|---|---|
| Phase A | | | |
| Cremophor® RH 40 | q.s. | PEG-40 Hydrogenated Castor Oil | BASF |
| Wasser demineralisiert | auf 100 | Aqua dem | |
| Phase B | | | |
| Luviquat® HM 552 | 10,00 | Polyquaternium-16 | BASF |
| 20 Gew.-%ige wässrige Lösung eines der Polymere aus S1 bis S10 | 0.5-5.0 | | |
| Luviquat® Mono CP | 0,50 | Hydroxyethyl Cetyldimonium Phosphate | BASF |
| Milchsäure oder Phosphorsäure | bis pH auf Wert im Bereich von 5 bis 6 | | |
| Phase C | | | |
| Propan/Butan | 6,00 | Propane/Butane | |

Herstellung: Einwiegen und unter Rühren Phasen A und B getrennt lösen und mischen. Phase B mit Milchsäure oder Phosphorsäure auf einen pH-Wert von pH 5 bis pH 6 einstellen, Phase A oder B gegebenenfalls noch weitere Stoffe wie Konservierungsmittel oder Parfümöl zusetzen. Abfüllen und Treibgas (Phase C) zusetzen.

SM78 bis SM88) Styling Mousse 8

| Inhaltsstoff | Gew.-% | Bezeichnung CTFA | Hersteller |
|---|---|---|---|
| Phase A | | | |
| Luviquat® Mono LS | 2,00 | Cocotrimonium Methosulfate | BASF |
| | | | |
| Phase B | | | |
| Hydagen HCMF | 2,00 | Chitosan | Cognis |
| 20 Gew.-%ige wässrige Lösung eines der Polymere aus S1 bis S10 | 0.5-5.0 | | |
| Dimethicone Copolyol | 0,50 | Dimethicone Copolyol | |
| Cremophor® A 25 | 0,20 | Ceteareth-25 | BASF |
| D-Panthenol USP® | 0,20 | Panthenol | BASF |
| Uvinul® P 25 | 0,10 | G-25 PABA | BASF |
| Milchsäure oder Phosphorsäure | bis pH auf Wert im Bereich von 5 bis 6 | | |
| Wasser demineralisiert | auf 100 | Aqua dem | |

(fortgesetzt)

| | | | |
|---|---|---|---|
| Phase C | | | |
| HFC 152 A | 10,00 | | . |

Herstellung : Einwiegen und unter Rühren Phasen A und B getrennt lösen und mischen. Phase B mit Milchsäure oder Phosphorsäure auf einen pH-Wert von pH 5 bis pH 6 einstellen, Phase A oder B gegebenenfalls noch weitere Stoffe wie Konservierungsmittel oder Parfümöl zusetzen. Abfüllen und Treibgas (Phase C) zusetzen.

SM89 bis SM99) Styling Mousse 9

| Inhaltsstoff | Gew.-% | Bezeichnung CTFA | Hersteller |
|---|---|---|---|
| Luviskol® VA 64 W | 10,00 | PVP/VA Copolymer | BASF |
| 20 Gew.-%ige wässrige Lösung eines der Polymere aus S1 bis S10 | 2.0-5.0 | | |
| Luviquat® Mono CP | 0,20 | Hydroxyethyl Cetyldimonium Phosphate | BASF |
| Dimethicone Copolyol | 0,50 | Dimethicone Copolyol | |
| Cremophor® A 25 | 0,20 | Ceteareth-25 | BASF |
| Ethanol | 10,00 | Alcohol | |
| Milchsäure oder Phosphorsäure | bis pH auf Wert im Bereich von 5 bis 6 | | |
| Wasser demineralisiert | auf 100 | Aqua dem | |
| Propan/Butan | 10,00 | Propane/Butane | |

Herstellung: Komponenten einwiegen und unter Rühren lösen und mischen. Mit Milchsäure oder Phosphorsäure auf einen pH-Wert von pH 5 bis pH 6 einstellen, gegebenenfalls noch weitere Stoffe wie Konservierungsmittel oder Parfümöl) zusetzen. Abfüllen und Treibgas zusetzen.

Verwendung in der Hautkosmetik:

C1 bis C11) Standard O/W-Creme

| Inhaltsstoff | Gew.-% | Bezeichnung CTFA | Hersteller |
|---|---|---|---|
| Ölphase | | | |
| Cremophor® A6 | 3.5 | Ceteareth-6 (and) Stearyl Alkohol | BASF |
| Cremophor® A25 | 3.5 | Ceteareth-25 | BASF |
| Glycerinmonostearat s.e. | 2.5 | Glyceryl Stearate | |
| Paraffinöl | 7.5 | Paraffin Oil | |
| Cetylalkohol | 2.5 | Cetyl Alkohol | |
| Luvitol® EHO | 3.2 | Cetearyl Octanoate | BASF |
| Vitamin-E-acetat | 1.0 | Tocopheryl Acetate | |

(fortgesetzt)

| Inhaltsstoff | Gew.-% | Bezeichnung CTFA | Hersteller |
|---|---|---|---|
| Nip-Nip | 0.1 | Methyl- und Propyl-4-hydroxybenzoate (7:3) | |
| Wasserphase | | | |
| Wasser demineralisiert | auf 50 | Aqua dem | |
| 20 Gew.-%ige wässrige Lösung eines | 2.0-8.0 | | |
| Inhaltsstoff | Gew.-% | Bezeichnung CTFA | Herstellen |
| der Polymere aus S1 bis S10 | | | |
| 1,2-Propylene Glycol Care | 1.5 | Propylene Glycol | BASF |
| Germall II | 0.1 | Imidazolidinyl-Urea | |
| Milchsäure oder Phosphorsäure | bis pH auf Wert im Bereich von 5 bis 6 | | |

Herstellung : Einwiegen und unter Rühren die Öl-Phasen und Wasser-Phase getrennt bei einer Temperatur von ca.80°C homogenisieren, wässrige Phase mit Milchsäure oder Phosphorsäure auf einen pH-Wert von pH 5 bis pH 6 einstellen. Wasser-Phase langsam in Öl-Phase einrühren. Unter Rühren langsam auf RT abkühlen .

T1 bis T11 Tageslotion

| Inhaltsstoff | Gew.-% | Bezeichnung CTFA | Hersteller |
|---|---|---|---|
| Ölphase | | | |
| Cremophor® A6 | 1.5 | Ceteareth-6 (and) Ceteareth-6 Stearyl Alkohol | BASF |
| Cremophor® A25 | 1.5 | Ceteareth-25 | BASF |
| Glycerinmonostearat s.e. | 5.0 | Glyceryl Stearate | |
| Uvinul® MS 40 | 0.5 | Benzophenone-4 | BASF |
| Paraffinöl | 3.5 | Paraffin Oil | |
| Cetylalkohol | 0.5 | Cetyl Alkohol | |
| Luvitol® EHO | 10.0 | Cetearyl Octanoate | BASF |
| D-Panthenol 50 P® | 3.0 | Panthenol und Pro-. pylenglykol | |
| Vitamin-E-acetate | 1.0 | Tocopheryl Acetate | |
| Tegiloxan® 100 | 0.3 | Dimethicone | |
| Nip-Nip | 0.1 | Methyl- und Propyl-4-hydroxybenzoate (7:3) | |
| Wasserphase | | | |
| 20 Gew.-%ige wässrige Lösung eines der Polymere aus S1 bis S10 | 2.5-5.0 | | |
| 1,2- Propylenglykol | 1.5 | Propylene Glycol | |
| Germall II | | Imidazolidinyl-Urea | |

(fortgesetzt)

| Inhaltsstoff | Gew.-% | Bezeichnung CTFA | Hersteller |
|---|---|---|---|
| Milchsäure oder Phosphorsäure | bis pH auf Wert im Bereich von 5 bis 6 | | |
| Wasser demineralisiert | auf 50 | Aqua dem | |

Herstellung : Einwiegen und unter Rühren die Öl-Phasen und Wasser-Phase getrennt bei einer Temperatur von ca. 80°C homogenisieren. Wasser-Phase mit Milchsäure oder Phosphorsäure auf einen pH-Wert von pH 5 bis pH 6 einstellen und langsam in Öl-Phase einrühren. Unter Rühren langsam auf RT abkühlen.

## Patentansprüche

**1.** Wässrige Zubereitung enthaltend

A) wenigstens ein wasserlösliches oder wasserdispergierbares Copolymer A) mit kationogenen Gruppen, das

a) wenigstens ein Monomer mit wenigstens einem protonierbaren Stickstoffatom ausgewählt aus

- wenigstens einer N-Vinylimidazol-Verbindung der allgemeinen Formel (I)

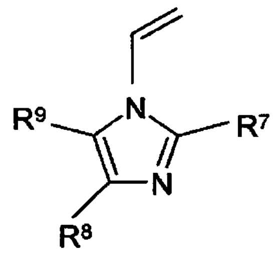

**(I)**

einpolymerisiert, worin $R^7$ bis $R^9$ unabhängig voneinander für Wasserstoff, $C_1$-$C_4$-Alkyl oder Phenyl stehen,
und

b) wenigstens ein N-Vinyllactam einpolymerisiert enthält, und

B) wenigstens einen kosmetisch akzeptablen Träger,
wobei der pH-Wert der wässrigen Zubereitung einen Wert im Bereich von pH 4 bis pH 6 aufweist.

**2.** Zubereitung nach Anspruch 1, wobei das Copolymer A) zusätzlich wenigstens ein von a) und b1) verschiedenes, damit copolymerisierbares nichtionisches wasserlösliches Monomer b2) ausgewählt unter

b2.1) N-Vinylamiden gesättigter $C_1$-$C_8$-Monocarbonsäuren,
b2.2) primären Amiden $\alpha,\beta$-ethylenisch ungesättigter Monocarbonsäuren und deren N-Alkyl- und N,N-Dialkylderivaten, die zusätzlich zu dem Carbonyl-Kohlenstoffatom der Amidgruppe höchstens 8 weitere Kohlenstoffatome aufweisen,
b2.3) Estern $\alpha,\beta$-ethylenisch ungesättigter Mono- und Dicarbonsäuren mit Diolen,
b2.4) Amiden $\alpha,\beta$-ethylenisch ungesättigter Mono- und Dicarbonsäuren mit Aminoalkoholen, die eine primäre oder sekundäre Aminogruppe aufweisen,
b2.5) Polyetheracrylaten und Mischungen daraus einpolymerisiert enthält.

**3.** Zubereitung nach den Ansprüchen 1 oder 2, wobei das Copolymer A) zusätzlich wenigstens ein Monomer b3), das

ausgewählt ist unter $\alpha,\beta$-ethylenisch ungesättigten wasserlöslichen Verbindungen mit kationischen hydrophilen Gruppen einpolymerisiert enthält.

**4.** Zubereitung nach Anspruch 3, wobei das Monomer b3) ausgewählt ist unter den Quatemisierungsprodukten von Monomer a).

**5.** Zubereitung nach den Ansprüchen 1 bis 4, wobei das Copolymer A)

a) 0.5 bis 40 Gew.-% N-Vinylimidazol und/oder eines Derivates davon,
b1) 20 bis 99.5 Gew.-% wenigstens eines N-Vinyllactams,
b2) 0 bis 50 Gew.-% wenigstens eines Monomers b2), welches ausgewählt ist unter
b2.1) N-Vinylamiden gesättigter $C_1$-$C_8$-Monocarbonsäuren,
b2.2) primären Amiden a,$\beta$-ethylenisch ungesättigter Monocarbonsäuren und deren N-Alkyl- und N,N-Dialkylderivaten, die zusätzlich zu dem Carbonyl-Kohlenstoffatom der Amidgruppe höchstens 8 weitere Kohlenstoffatome aufweisen,
b2.3) Estern $\alpha,\beta$-ethylenisch ungesättigter Mono- und Dicarbonsäuren mit Diolen,
b2.4) Amiden $\alpha,\beta$-ethylenisch ungesättigter Mono- und Dicarbonsäuren mit Aminoalkoholen, die eine primäre oder sekundäre Aminogruppe aufweisen,
b2.5) Polyetheracrylaten und Mischungen davon und
b3) 0 bis 30 Gew.-% wenigstens eines Monomers, das ausgewählt ist unter $\alpha,\beta$-ethylenisch ungesättigten wasserlöslichen Verbindungen mit kationischen hydrophilen Gruppen, einpolymerisiert enthält, mit der Maßgabe, dass die Summe der Mengen der Komponenten a) bis b3) 100 Gew.-% ergibt.

**6.** Zubereitung nach Anspruch 5, wobei das Copolymer A)

a) 1 bis 30 Gew.-% des Monomeren a),
b1) 20 bis 70 Gew.-% des Monomeren b1),
b2) 5 bis 50 Gew.-% des Monomeren b2) und
b3) 0 bis 30 Gew.-% des Monomeren b3)

einpolymerisiert enthält, mit der Maßgabe, dass die Summe der Mengen der Komponenten a) bis b3) 100 Gew.-% ergibt.

**7.** Zubereitung nach Anspruch 6, wobei das Copolymer A)

a) 3 bis 20 Gew.-% des Monomeren a),
b1) 30 bis 70 Gew.-% des Monomeren b1),
b2) 10 bis 40 Gew.-% des Monomeren b2), und
b3) 0 bis 10 Gew.-% des Monomeren b3)

einpolymerisiert enthält mit der Maßgabe, dass die Summe der Mengen der Komponenten a) bis b3) 100 Gew.-% ergibt.

**8.** Zubereitung nach einem der Ansprüche 5 bis 7, wobei das Copolymer A)

a) N-Vinylimidazol,
b1) N-Vinylpyrrolidon,
b2) gegebenenfalls wenigstens ein Monomer b2)
b3) gegebenenfalls wenigstens ein Monomer b3), das ausgewählt ist unter Quaternisierungsprodukten des N-Vinylimidazols und des Dimethylaminopropylmethacrylsäureamids,

einpolymerisiert enthält.

**9.** Zubereitung nach einem der der Ansprüche 1 bis 8, wobei das Copolymer A)

a) 3 bis 15 Gew.-% N-Vinylimidazol
b1) 30 bis 70 Gew.-% N-Vinylpyrrolidon
b2) 20 bis 35 Gew.-% Methacrylsäureamid

b3) 0 bis 10 Gew.-% quatemiertes N-Vinylimidazol

einpolymerisiert enthält mit der Maßgabe, dass die Summe der Mengen der Komponenten a) bis b3) 100 Gew.-% ergibt.

**10.** Zubereitung nach einem der der Ansprüche 1 bis 9, wobei die Komponente B) ausgewählt ist unter
i Wasser,
ii wassermischbaren organischen Lösungsmitteln, vorzugsweise $C_2$-$C_4$-Alkanolen, insbesondere Ethanol,
iii Ölen, Fetten, Wachsen,
iv von iii) verschiedenen Estern von $C_6$-$C_{30}$-Monocarbonsäuren mit ein-, zwei- oder dreiwertigen Alkoholen,
v gesättigten acyclischen und cyclischen Kohlenwasserstoffen,
vi Fettsäuren,
vii Fettalkoholen,
viii Treibgasen
und Mischungen davon.

**11.** Zubereitung nach einem der der Ansprüche 1 bis 10, enthaltend wenigstens einen von den Komponenten A) und B) verschiedenen Zusatzstoff, der ausgewählt ist unter kosmetisch aktiven Wirkstoffen, Emulgatoren, Tensiden, Konservierungsmitteln, Parfümölen, Verdickern, Haarpolymeren, Haar- und Hautconditionem, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettem, Collagen, Eiweisshydrolysaten, Lipiden, Antioxidantien, Entschäumern, Antistatika, Emollienzien und Weichmachern.

**12.** Zubereitung nach einem der der Ansprüche 1 bis 11, enthaltend wenigstens ein weiteres wasserlösliches Polymer.

**13.** Zubereitung nach Anspruch 12, wobei das wasserlösliche Polymer Chitosan und/oder ein Chitosanderivat ist.

**14.** Zubereitung nach einem der der Ansprüche 1 bis 13 in Form eines Gels, Schaums, Sprays, einer Mousse, Salbe, Creme, Emulsion, Suspension, Lotion, Milch oder Paste.

**15.** Zubereitung nach einem der Ansprüche 1 bis 14, die zusätzlich wenigstens ein nichtionisches Verdickungsmittel enthält.

**16.** Zubereitung nach einem der Ansprüche 1 bis 15, wobei der pH Wert einen Wert im Bereich von pH 5 bis pH 6 aufweist.

**17.** Zubereitung nach einem der Ansprüche 1 bis 16, wobei der pH-Wert durch Zugabe von Hydroxycarbonsäure eingestellt wird.

**18.** Zubereitung nach einem der Ansprüche 1 bis 16, wobei der pH-Wert durch Zugabe von anorganischer Säure eingestellt wird.

**19.** Verfahren zur Herstellung einer wässrigen Zubereitung nach den Ansprüchen 1 bis 18, **dadurch gekennzeichnet, dass** die Einstellung des pH Wertes nach abgeschlossener Herstellung von Komponente A) vorgenommen wird.

**20.** Verwendung einer Zubereitung nach einem der Ansprüche 1 bis 18 in Hautreinigungsmitteln, Mitteln zur Pflege und zum Schutz der Haut, Nagelpflegemitteln, Zubereitungen für die dekorative Kosmetik und Haarbehandlungsmitteln.

**21.** Verwendung nach Anspruch 20 in Haarbehandlungsmitteln als Festiger und/oder als Conditioner.

**22.** Verwendung nach den Ansprüchen 20 oder 21, wobei das Mittel in Form eines Haargels, Shampoos, Schaumfestigers, Haarwassers, Haarsprays oder Haarschaums vorliegt.

## Claims

**1.** An aqueous preparation comprising

A) at least one water-soluble or water-dispersible copolymer A) with cationogenic groups which comprises

a) at least one monomer with at least one protonatable nitrogen atom chosen from

- at least one N-vinylimidazole compound of the general formula (I)

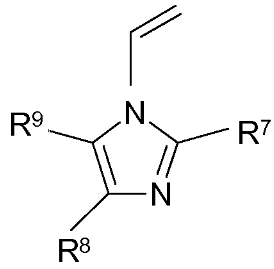

(I)

in copolymerized form, in which $R^7$ to $R^9$, independently of one another, are hydrogen, $C_1$-$C_4$-alkyl or phenyl,
and

b) at least one N-vinyllactam in copolymerized form, and

B) at least one cosmetically acceptable carrier,
where the pH of the aqueous preparation has a value in the range from pH 4 to pH 6.

**2.** The preparation according to claim 1, where the copolymer A) additionally comprises at least one nonionic water-soluble monomer b2) which is different from a) and b1) and copolymerizable therewith, chosen from
b2.1) N-vinylamides of saturated $C_1$-$C_8$-monocarboxylic acids,
b2.2) primary amides of α,β-ethylenically unsaturated monocarboxylic acids and their N-alkyl and N,N-dialkyl derivatives which, in addition to the carbonyl carbon atom of the amide group, have at most 8 further carbon atoms,
b2.3) esters of α,β-ethylenically unsaturated mono-and dicarboxylic acids with diols,
b2.4) amides of α,β-ethylenically unsaturated mono-and dicarboxylic acids with aminoalcohols which have a primary or secondary amino group,
b2.5) polyether acrylates and mixtures thereof in copolymerized form.

**3.** The preparation according to claims 1 and 2, where the copolymer A) additionally comprises at least one monomer b3) which is chosen from α,β-ethylenically unsaturated water-soluble compounds with cationic hydrophilic groups in copolymerized form.

**4.** The preparation according to claim 3, where the monomer b3) is chosen from the quaternization products of monomer a).

**5.** The preparation according to claims 1 to 4, where the copolymer A) comprises

a) 0.5 to 40% by weight of N-vinylimidazole and/or a derivative thereof,
b1) 20 to 99.5% by weight of at least one N-vinyllactam,
b2) 0 to 50% by weight of at least one monomer b2) which is chosen from
b2.1) N-vinylamides of saturated $C_1$-$C_8$-monocarboxylic acids,
b2.2) primary amides of α,β-ethylenically unsaturated monocarboxylic acids and their N-alkyl and N,N-dialkyl derivatives which, in addition to the carbonyl carbon atom of the amide group, have at most 8 further carbon atoms,
b2.3) esters of α,β-ethylenically unsaturated mono-and dicarboxylic acids with diols,
b2.4) amides of α,β-ethylenically unsaturated mono-and dicarboxylic acids with aminoalcohols which have a primary or secondary amino group,
b2.5) polyether acrylates and mixtures thereof and
b3) 0 to 30% by weight of at least one monomer which is chosen from α,β-ethylenically unsaturated water-soluble compounds with cationic hydrophilic groups, in copolymerized form,
with the proviso that the sum of the amounts of components a) to b3) is 100% by weight.

**6.** The preparation according to claim 5, where the copolymer A) comprises

a) 1 to 30% by weight of monomer a),
b1) 20 to 70% by weight of monomer b1),
b2) 5 to 50% by weight of monomer b2) and
b3) 0 to 30% by weight of monomer b3)

in copolymerized form, with the proviso that the sum of the amounts of components a) to b3) is 100% by weight.

**7.** The preparation according to claim 6, where the copolymer A) comprises

a) 3 to 20% by weight of monomer a),
b1) 30 to 70% by weight of monomer b1),
b2) 10 to 40% by weight of monomer b2), and
b3) 0 to 10% by weight of monomer b3)

in copolymerized form, with the proviso that the sum of the amounts of components a) to b3) is 100% by weight.

**8.** The preparation according to one of claims 5 to 7, where the copolymer A) comprises

a) N-vinylimidazole,
b1) N-vinylpyrrolidone,
b2) optionally at least one monomer b2)
b3) optionally at least one monomer b3) which is chosen from quaternization products of N-vinylimidazole and of dimethylaminopropyl-methacrylamide,

in copolymerized form.

**9.** The preparation according to one of claims 1 to 8, where the copolymer A) comprises

a) 3 to 15% by weight of N-vinylimidazole
b1) 30 to 70% by weight of N-vinylpyrrolidone
b2) 20 to 35% by weight of methacrylamide
b3) 0 to 10% by weight of quaternized N-vinylimidazole

in copolymerized form, with the proviso that the sum of the amounts of components a) to b3) is 100% by weight.

**10.** The preparation according to one of claims 1 to 9, where the component B) is chosen from
i water,
ii water-miscible organic solvents, preferably $C_2$-$C_4$-alkanols, in particular ethanol,
iii oils, fats, waxes,
iv esters of $C_6$-$C_{30}$-monocarboxylic acids with mono-, di- or trihydric alcohols which are different from iii),
v saturated acyclic and cyclic hydrocarbons,
vi fatty acids,
vii fatty alcohols,
viii propellant gases
and mixtures thereof.

**11.** The preparation according to one of claims 1 to 10, comprising at least one additive different from components A) and B) which is chosen from cosmetically active ingredients, emulsifiers, surfactants, preservatives, perfume oils, thickeners, hair polymers, hair and skin conditioners, graft polymers, water-soluble or dispersible silicone-containing polymers, light protection agents, bleaches, gel formers, care agents, colorants, tinting agents, tanning agents, dyes, pigments, consistency-imparting agents, humectants, refatting agents, collagen, protein hydrolyzates, lipids, antioxidants, antifoams, antistats, emollients and softeners.

**12.** The preparation according to one of claims 1 to 11, comprising at least one further water-soluble polymer.

**13.** The preparation according to claim 12, where the water-soluble polymer is chitosan and/or a chitosan derivative.

**14.** The preparation according to one of claims 1 to 13 in the form of a gel, foam, spray, mousse, ointment, cream, emulsion, suspension, lotion, milk or paste.

**15.** The preparation according to one of claims 1 to 14 which additionally comprises at least one nonionic thickener.

**16.** The preparation according to one of claims 1 to 15, where the pH has a value in the range from pH 5 to pH 6.

**17.** The preparation according to one of claims 1 to 16, where the pH is adjusted by adding hydroxycarboxylic acid.

**18.** The preparation according to one of claims 1 to 16, where the pH is adjusted by adding inorganic acid.

**19.** A method of preparing an aqueous preparation according to claims 1 to 18, wherein the pH adjustment takes place after the preparation of component A) is complete.

**20.** The use of a preparation according to one of claims 1 to 18 in skin-cleansing compositions, compositions for the care and the protection of the skin, nailcare compositions, preparations for decorative cosmetics and hair-treatment compositions.

**21.** The use according to claim 20 in hair-treatment compositions as setting agent and/or as conditioner.

**22.** The use according to claims 20 or 21, where the composition is in the form of a hair gel, shampoo, setting foam, hair tonic, hairspray or hair foam.

**Revendications**

**1.** Composition aqueuse contenant

A) au moins un copolymère A) soluble ou dispersible dans l'eau présentant des groupes cationogènes, qui contient, sous forme copolymérisée,

a) au moins un monomère présentant au moins un atome d'azote protonable, choisi parmi

- au moins un composé de N-vinylimidazole
de formule générale (I)

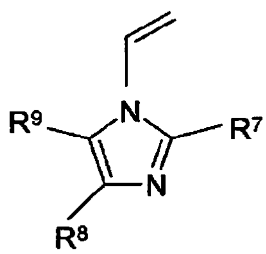

**(I)**

dans laquelle $R^7$ à $R^9$ représentent, indépendamment l'un de l'autre, hydrogène, $C_1$-$C_4$-alkyle ou phényle,

et
b) au moins un N-vinyllactame b1), et

B) au moins un support cosmétiquement acceptable, le pH de la préparation aqueuse présentant une valeur

dans la plage de pH 4 à pH 6.

2. Préparation selon la revendication 1, le copolymère A) contenant en outre, sous forme copolymérisée, au moins un monomère b2) différent de a) et de b1), copolymérisable avec ceux-ci, non ionique, soluble dans l'eau, choisi parmi

b2.1) les N-vinylamides d'acides $C_1$-$C_8$-monocarboxyliques saturés,
b2.2) les amides primaires d'acides monocarboxyliques $\alpha$,ß-éthyléniquement insaturés et leurs dérivés N-alkyle et N,N-dialkyle, qui présentent, en plus de l'atome de carbone carbonyle du groupe amide, au plus 8 autres atomes de carbone,
b2.3) les esters d'acides monocarboxyliques et dicarboxyliques $\alpha$,ß-éthyléniquement insaturés avec des diols
b2.4) les amides d'acides monocarboxyliques et dicarboxyliques $\alpha$,ß-éthyléniquement insaturés avec des aminoalcools, qui présentent un groupe amino primaire ou secondaire,
b2.5) les polyétheracrylates et les mélanges de ceux-ci.

3. Préparation selon les revendications 1 ou 2, le copolymère A) contenant en outre, sous forme copolymérisée au moins un monomère b3), qui est choisi parmi les composés $\alpha$,ß-éthyléniquement insaturés, solubles dans l'eau présentant des groupes cationiques hydrophiles.

4. Préparation selon la revendication 3, le monomère b3) étant choisi parmi les produits de quaternisation du monomère a).

5. Préparation selon les revendications 1 à 4, le copolymère A) contenant, sous forme copolymérisée,

a) 0,5 à 40 % en poids de N-vinylimidazole et/ou d'un dérivé de celui-ci,
b1) 20 à 99,5% en poids d'au moins un N-vinyllactame,
b2) 0 à 50% en poids d'au moins un monomère b2), qui est choisi parmi
b2.1) les N-vinylamides d'acides $C_1$-$C_8$-monocarboxyliques saturés,
b2.2) les amides primaires d'acides monocarboxyliques $\alpha$,ß-éthyléniquement insaturés et leurs dérivés N-alkyle et N,N-dialkyle, qui présentent, en plus de l'atome de carbone carbonyle du groupe amide, au plus 8 autres atomes de carbone,
b2.3) les esters d'acides monocarboxyliques et dicarboxyliques $\alpha$,ß-éthyléniquement insaturés avec des diols
b2.4) les amides d'acides monocarboxyliques et dicarboxyliques $\alpha$,ß-éthyléniquement insaturés avec des aminoalcools, qui présentent un groupe amino primaire ou secondaire,
b2.5) les polyétheracrylates et les mélanges de ceux-ci et
b3) 0 à 30% en poids d'au moins un monomère qui est choisi parmi les composés $\alpha$,ß-éthyléniquement insaturés solubles dans l'eau présentant des groupes cationiques hydrophiles, sous réserve que la somme des quantités des composants a) à b3) vaut 100% en poids.

6. Préparation selon la revendication 5, le copolymère A) contenant, sous forme copolymérisée,

a) 1 à 30% en poids du monomère a),
b1) 20 à 70% en poids du monomère b1),
b2) 5 à 50% en poids du monomère b2) et
b3) 0 à 30% en poids du monomère b3)

sous réserve que la somme des quantités des composants a) à b3) vaut 100% en poids.

7. Préparation selon la revendication 6, le copolymère A) contenant, sous forme copolymérisée,

a) 3 à 20% en poids du monomère a),
b1) 30 à 70% en poids du monomère b1),
b2) 10 à 40% en poids du monomère b2) et
b3) 0 à 10% en poids du monomère b3)

sous réserve que la somme des quantités des composants a) à b3) vaut 100% en poids.

8. Composition selon l'une quelconque des revendications 5 à 7, le copolymère A) contenant, sous forme copolymérisée,

a) du N-vinylimidazole,
b1) de la N-vinylpyrrolidone,
b2) le cas échéant au moins un monomère b2)
b3) le cas échéant au moins un monomère b3), qui est choisi parmi les produits de quaternisation du N-vinylimidazole et de l'amide de l'acide diméthylaminopropylméthacrylique.

**9.** Composition selon l'une quelconque des revendications 1 à 8, le copolymère A) contenant, sous forme copolymérisée,

a) 3 à 15 % en poids de N-vinylimidazole
b1) 30 à 70 % en poids de N-vinylpyrrolidone
b2) 20 à 35% en poids de méthacrylamide
b3) 0 à 10% en poids de N-vinylimidazole quaternisé sous réserve que la somme des quantités des composants a) à b3) vaut 100% en poids.

**10.** Composition selon l'une quelconque des revendications 1 à 9, le composant B) étant choisi parmi
i l'eau,
ii les solvants organiques miscibles à l'eau, de préférence les $C_2$-$C_4$-alcanols, en particulier l'éthanol,
iii les huiles, les graisses, les cires,
iv les esters différents de iii) d'acides monocarboxyliques en $C_6$-$C_{30}$ avec des alcools monovalents, divalents ou trivalents,
v les hydrocarbures acycliques et cycliques saturés,
vi les acides gras,
vii les alcools gras,
viii les gaz propulseurs
et leurs mélanges.

**11.** Préparation selon l'une quelconque des revendications 1 à 10, contenant au moins un additif différent des composants A) et B), qui est choisi parmi les substances cosmétiquement actives, les émulsifiants, les agents tensioactifs, les conservateurs, les huiles parfumées, les épaississants, les polymères pour cheveux, les revitalisants pour cheveux et la peau, les polymères greffés, les polymères contenant du silicone solubles dans l'eau ou dispersibles, les agents de protection contre la lumière, les agents de blanchiment, les gélifiants, les agents de soin, les teintures, les agents de coloration, les agents de bronzage, les colorants, les pigments, les agents conférant une consistance, les humectants, les relipidants, le collagène, les hydrolysats de protéines, les lipides, les antioxydants, les antimousses, les antistatiques, les émollients et les plastifiants.

**12.** Préparation selon les revendications 1 à 11, contenant au moins un autre polymère soluble dans l'eau.

**13.** Préparation selon la revendication 12, le polymère soluble dans l'eau étant le chitosane et/ou un dérivé de chitosane.

**14.** Préparation selon l'une quelconque des revendications 1 à 13 sous forme d'un gel, d'une mousse, d'un spray, d'une pommade, d'une crème, d'une émulsion, d'une suspension, d'une lotion, d'un lait ou d'une pâte.

**15.** Préparation selon l'une quelconque des revendications 1 à 14, qui contient en outre au moins un épaississant non ionique.

**16.** Préparation selon l'une quelconque des revendications 1 à 15, le pH présentant une valeur dans la plage de pH 5 à pH 6.

**17.** Préparation selon l'une quelconque des revendications 1 à 16, le pH étant réglé par addition d'acide hydroxycarboxylique.

**18.** Préparation selon l'une quelconque des revendications 1 à 16, le pH étant réglé par addition d'acide inorganique.

**19.** Procédé pour la préparation d'une préparation aqueuse selon les revendications 1 à 18, **caractérisé en ce que** le réglage du pH est réalisé après la fin de la préparation du composant A).

**20.** Utilisation d'une composition selon l'une quelconque des revendications 1 à 18 dans des agents de nettoyage de

la peau, des agents pour le soin et la protection de la peau, des agents de soin des ongles, des préparations pour la cosmétique décorative et des agents de traitement des cheveux.

**21.** Utilisation selon la revendication 20 dans les agents de traitement des cheveux comme fixateurs et/ou comme revitalisants.

**22.** Utilisation selon les revendications 20 ou 21, l'agent se trouvant sous forme d'un gel pour cheveux, d'un shampooing, d'un fixateur en mousse, d'une lotion capillaire, d'un spray pour cheveux ou d'une mousse pour cheveux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 670333 A
- EP 929285 A
- WO 0027893 A
- WO 0392640 A
- DE 19838851
- WO 03092640 A
- DE 4333238 A
- DE 3929973
- DE 2150557
- DE 2817369
- DE 3708451
- US 3405084 A
- EP 0257444 A
- EP 0480280 A
- DE 4223066 A
- EP 636361 A
- WO 9725021 A
- EP 751162 A
- DE 4225045 A


### In der Beschreibung aufgeführte Nicht-Patentliteratur


- *Cosmetic & Toiletries,* 1988, vol. 103, 23
- Ullmann's Encyclopedia of Industrial Chemistry. vol. 17, 645-655
- **KARL-HEINZ SCHRADER.** Grundlagen und Rezepturen der Kosmetika. Verlag Hüthig, 319-355
- **FIEDLER, H. P.** Lexikon der Hilfsstoffe für Pharmazie, Kosmetik und angrenzende Gebiete. ECV-Editio-Kantor-Verlag, 1996
- Grundlagen und Rezepturen der Kosmetika. **SCHRADER.** Hüthig Buch. Verlag, Heidelberg, 1989
- Die K-Werte werden nach Fikentscher. *Cellulosechemie,* 1932, vol. 13, 58-64